# EUROPÄISCHE PATENTSCHRIFT

(11) **EP 2 721 054 B1**
(45) Veröffentlichungstag und Bekanntmachung des Hinweises auf die Patenterteilung: **03.10.2018**
(21) Anmeldenummer: 12730462.4
(22) Anmeldetag: 20.06.2012
(51) Int. Cl.: C07K 7/08, C12Q 1/37, A01N 37/46, A01N 63/02, A61K 38/10, C07K 14/435

(54) **MODIFIZIERTE ANTIBIOTISCHE PEPTIDE MIT VARIABLER SYSTEMISCHER FREISETZUNG**
MODIFIED ANTIBIOTIC PEPTIDES HAVING VARIABLE SYSTEMIC RELEASE
PEPTIDES ANTIBIOTIQUES MODIFIÉS À LIBÉRATION SYSTÉMIQUE VARIABLE

(30) Priorität: 20.06.2011 DE 102011118029
(43) Veröffentlichungstag der Anmeldung: 23.04.2014
(73) Patentinhaber: Universität Leipzig, 04109 Leipzig (DE)
(72) Erfinder: HOFFMANN, Ralf, 04463 Großpösna (DE); BERTHOLD, Nicole, 04279 Leipzig (DE); NOLLMANN, Friederike, 60433 Frankfurt am Main (DE)
(74) Vertreter: Kailuweit & Uhlemann Patentanwälte Partnerschaft mbB
(86) Internationale Anmeldenummer: PCT/EP2012/061780
(87) Internationale Veröffentlichungsnummer: WO 2012/175532

(56) Entgegenhaltungen:
- WO-A1-2009/013262
- WO-A2-2010/072752
- DE-A1-102009 007 381
- HONGJIAN LI ET AL: "A Protease-Based Strategy for the Controlled Release of Therapeutic Peptides", ANGEWANDTE CHEMIE (INTERNATIONAL ED. IN ENGLISH), Bd. 122, Nr. 29, 5. Juli 2010 (2010-07-05) , Seiten 5050-5053, XP55011884, ISSN: 0044-8249, DOI: 10.1002/ange.201000287 in der Anmeldung erwähnt
- FRANCESCO M VERONESE ET AL: "PEG-Doxorubicin Conjugates: Influence of Polymer Structure on Drug Release, in Vitro Cytotoxicity, Biodistribution, and Antitumor Activity", BIOCONJUGATE CHEMISTRY, Bd. 16, Nr. 4, 1. Juli 2005 (2005-07-01), Seiten 775-784, XP55036563, ISSN: 1043-1802, DOI: 10.1021/bc040241m
- BENEDICT LAW ET AL: "Proteolysis: A Biological Process Adapted in Drug Delivery, Therapy, and Imaging", BIOCONJUGATE CHEMISTRY, Bd. 20, Nr. 9, 16. September 2009 (2009-09-16), Seiten 1683-1695, XP55036460, ISSN: 1043-1802, DOI: 10.1021/bc800500a in der Anmeldung erwähnt
- FILPULA ET AL: "Releasable PEGylation of proteins with customized linkers", ADVANCED DRUG DELIVERY REVIEWS, ELSEVIER BV, AMSTERDAM, NL, Bd. 60, Nr. 1, 30. November 2007 (2007-11-30), Seiten 29-49, XP022370570, ISSN: 0169-409X, DOI: 10.1016/J.ADDR.2007.02.001
- SIMONA JEVEVAR ET AL: "PEGylation of therapeutic proteins", BIOTECHNOLOGY JOURNAL, WILEY-VCH VERLAG, WEINHEIM, DE, vol. 5, no. 1, 1 January 2010 (2010-01-01) , pages 113-128, XP002591544, ISSN: 1860-6768, DOI: 10.1002/BIOT.200900218 [retrieved on 2010-01-12]

## Beschreibung

Diese Erfindung betrifft modifizierte antibiotische Peptide insbesondere für die Verwendung in der Medizin. Weiterhin bezieht sich die Erfindung auf Zusammensetzungen und Methoden zum Abtöten von Mikroorganismen, wie Bakterien, Viren oder Pilze, und Methoden zur Behandlung mikrobieller Infektionen.

Das Auftreten ernsthafter Bakterien- und Pilzinfektionen ist ein steigendes Problem trotz bemerkenswerter Fortschritte in der Antibiotikatherapie. Jedes Jahr gibt es mehr als 40 Millionen Krankenhausaufenthalte in den Vereinigten Staaten von Amerika und mehr als 2 Millionen dieser Patienten infizieren sich im Krankenhaus. In 50-60% dieser Fälle sind Antibiotika-resistente Bakterien involviert. Diese im Krankenhaus erworbenen Krankheiten führen zu schätzungsweise 60.000-70.000 Todesfällen in den USA und bis zu 10.000 Todesfällen in Deutschland.

Dies verdeutlicht die Notwendigkeit der weiteren Suche nach neuen Antibiotika. Induzierbare antibakterielle Peptide repräsentieren ein Forschungsfeld, in dem die heutige Biochemie, Immunologie und Wirkstoffforschung zusammentreffen. Peptidantibiotika, mit einer Größe von 13 bis zu mehr als hundert Aminosäuren, wurden aus Pflanzen, Tieren und Mikroben isoliert (Boman, H.G. 1995 Annu. Rev. Immunol. 13: 61-92).

Ein einzelnes Tier besitzt ca. 6-10 antimikrobielle Peptide, wobei jedes Peptid oft ein komplett anderes Aktivitätsspektrum zeigt (Barra, Det al. 1998. FEBS Lett. 430: 130-134). Es ist bekannt, dass die überwältigende Anzahl an antibakteriellen Peptiden einschließlich den gut untersuchten Defensinen, Cecropinen und Magaininen, durch einen "lytischen/ionischen" Mechanismus wirken. Als gemeinsamen Wirkmechanismus dieser "lytischen" Peptide wird ein permeabilisierender Effekt auf die bakterielle Zytoplasmamembran diskutiert. Eine kationische, amphipathische Struktur, die hydrophile Ionen-(Protonen-) Kanäle in einer Lipiddoppelschicht ausbildet, ist Grundlage dieser Aktivität. Durch das Austreten von Ionen wird das für viele grundlegende Lebensprozesse notwendige Membranpotential zerstört und so die Zelle abgetötet. Diese lytischen Peptide wirken oft in höheren Konzentrationen toxisch auf Säugetiermembranen, was ihre Eignung als mögliche Arzneimittel einschränkt. Wird Prolin in die Sequenz der α-helikalen antimikrobiellen Peptide eingefügt, so sinkt, in Abhängigkeit von der Anzahl der Prolin-Reste, die Fähigkeit der Peptide die Cytoplasmamembran von *E. coli* zu permeabilisieren. Bei dieser Betrachtung ist es verblüffend, dass einige der aktivsten, nativen antibakteriellen Peptide, zumindest in Bezug auf einige Gram-negative Pathogene, zu der Familie der Prolin-reichen Peptide gehören (Otvos, L. et al. 2000. Protein Sci. 9: 742-749).

Die oben beschriebenen Nebeneffekte könnten durch antimikrobielle Peptide (AMP) überwunden werden, die ein bakterielles Protein oder andere intra- oder extrazelluläre Komponenten spezifisch erkennen, ohne eine Kreuzreaktivität mit Säugetieranaloga zu zeigen. Dies scheint auf Prolin-reiche antimikrobielle Peptide, einschließlich Apidaecine, Drosocin und Pyrrhocoricin die ursprünglich aus Insekten isoliert wurden, zuzutreffen. Mit der enormen Variation in der Größe und in den biochemischen Eigenschaften, ist es nicht überraschend, dass die Struktur-Wirkungs- und Konformations-Wirkungs-Beziehungen der Fokus der antibakteriellen Peptidforschung ist. Eine komplette Untersuchung des natürlichen, antibakteriellen Peptidrepertoires auf die biologische Stärke ist nicht nur für generelle biochemische Fragestellungen wichtig, sondern auch für die pharmazeutische Industrie von anhaltendem Interesse. Trotz der Probleme von *in vitro* Tests mit Peptid-basierenden Antibiotika, haben einige natürliche, kationische antibakterielle Peptide schon die klinische Testphase erreicht (Boman, H.G. 1995 ebd.). Während einige dieser Peptide als topische (örtlich) Mittel in der frühen klinischen Testphase Wirkung zeigten, waren andere in der systemischen Therapie aktiv. Zum Beispiel hat das kationische Protein rBPI 21, das zur parentalen Behandlung von Meningococcaemia eingesetzt wird, die dritte Phase der klinischen Prüfung abgeschlossen (Boman, H.G. 1995 ebd.).
Die Familie der Prolin-reichen Peptide (z.B. Apidaecin, Drosocin und Pyrrhocoricin) töten Bakterien nicht durch Permeabilisierung ihrer Membran, sondern binden stereospezifisch an ein oder mehrere Zielproteine. Diese möglichen Interaktionspartner, bisher wurde das Hitzeschock Protein DnaK gut untersucht (u. a. Boman, H.G. 1995), werden durch die Prolin-reichen Peptide inhibiert und vermutlich die korrekte Proteinfaltung verhindert, was letztendlich zum Zelltod führt. Zudem scheinen Prolin-reiche Peptide, im starken Gegensatz zu AMPs mit definierter Sekundärstruktur wie Melittin oder Gramicidin, *in vitro* weder hämolytisch noch toxisch auf eukaryotische Zellen zu wirken. Entscheidenden Einfluss auf die Entwicklung neuer peptidbasierter Antibiotika hat neben der antimikrobiellen Aktivität vor allem die Stabilität in Säugetierserum (25%). So wird beispielsweise Drosocin innerhalb einer Stunde abgebaut, während Pyrrhocoricin mit Halbwertszeiten von 120 Minuten erheblich stabiler gegenüber Proteasen ist. Dabei werden vermutlich nicht nur die N- und C-Termini durch Amino- und Carboxypeptidasen abgespalten, sondern die Peptide auch durch Endoproteasen verdaut. Die dabei entstehenden Metaboliten sind teilweise stabil gegenüber weiterem Abbau, verlieren allerdings meist die antimikrobielle Aktivität (MHK-Werten ≥ 64 µg/mL).

In Experimenten von Schneider M. und Dorn A. (2001. J Invertebr Pathol. 78: 135-40) wurden Nymphen und Puppen der Milchkrautwanze *Oncopeltus fasciatus* aus der Familie der *Lygaeidae* mit zwei verschiedenen gram-negativen *Pseudomonas* Spezies infiziert und deren Immunantwort analysiert. Während eine Infektion der Nymphen von *O*. *fasciatus* mit dem humanen Pathogen *Pseudomonas aeruginosa,* nach 48 h den Tod aller Individuen zur Folge hatte, überlebten 71% der mit weniger pathogenen *Pseudomonas putida* infizierten Individuen mindestens 96 h. Wurden die Nymphen der Milchkrautwanze nun zuerst mit *P. putida* und nach 24 h mit *P. aeruginosa* infiziert, stieg hier die Überlebensrate der doppelt infizierten Individuen innerhalb der ersten 24 h signifikant auf 73%. Die wahrscheinliche Induzierung der Synthese von antibakteriellen Peptiden, durch die sich Insekten im Rahmen ihres angeborenen Immunsystems gegen eindringende Mikroorganismen wehren, wurde anschließend untersucht. Vier Peptide (Oncopeltus antibakterielles Peptid 1-4) wurden mit Molekulargewichten von 15, 8, 5 bzw. 2 kDa identifiziert und für die antibakterielle Wirkung verantwortlich gemacht. Die Sequenzanalyse nach Edman ergab neben einer 34 Aminosäure langen Teilsequenz für Peptid 1 (15 kDa) auch die unvollständige Sequenz des Prolin-reichen 2 kDa Peptids 4. Es war nicht möglich die Aminosäuren an Positionen 11 und die C-terminale Sequenz ab Position 19 eindeutig zu identifizieren. Das genaue Molekulargewicht ist unbekannt.

Eine Auswahl bisher bekannter Sequenzen von antibiotischen Peptiden ist in Tabelle 1 aufgelistet:

**Tabelle 1:**

| Peptid | Species | Sequenz | SEQ ID Nr. |
|---|---|---|---|
| Apidaecin 1a | *Apis mellifera* | GNNRPVYIPQPRPPHPRI | 1 |
| Apidaecin Ib | *Apis mellifera* | GNNRPVYIPQPRPPHPRL | 2 |
| Drosocin | *Drosophila melanogaster* | GKPRPYSPRPTSHPRPIRV | 3 |
| Formaecin 1 | *Myrmecia gulosa* | GRPNPVNNKPTPYPHL | 4 |
| Pyrrhocoricin | *Pyrrhocoris apterus* | VDKGSYLPRPTPPRPIYNRN-NH₂ | 5 |
| Metalnikowin 1 | *Palomena prasina* | VDKPDYRPRPRPPNM | 6 |
| Oncopeltus antibakterielles Peptid 1 | *Oncopeltus fasciatus* | | 7 |
| Oncopeltus antibakterielles Peptid 4 | *Oncopeltus fasciatus* | VDKPPYLPRP(X/P)PPRRIYN(NR) | 8 |

Apidaecinderivate sind in WO2009013262A1 offenbart. Derivate des Oncopeltus antibakteriellen Peptid 4 sind in WO2010086401A1 und DE102009007381A1 offenbart.

In der Literatur werden unterschiedliche Ansätze beschrieben, um die pharmakokinetischen Eigenschaften von pharmakologischen Wirkstoffen zu beeinflussen. Dabei kommen auch organische Polymere (wie z. B. Polyethylenglykol) zur Anwendung.

Die Verwendung von Polyethylenglykol (PEG) in pharmazeutischen Darreichungsformen zur kontrollierten Feisetzung eines Wirkstoffes ist bekannt. Hierbei müssen zwei Formen unterschieden werden:
1. Das Einbringen des Wirkstoffes in ein vernetztes PEG-Hydrogel;
2. Die direkte Anbindung eines linearen oder verzweigten PEG-Moleküls an den Wirkstoff (sogenannte PEGylierung).

Beide Formen unterscheiden sich nicht nur in der Pharmakokinetik, sondern vor allem den möglichen Administrationsrouten. Das Hydrogel wird lokal verabreicht. Der PEGylierte Wirkstoff systemisch (in der Regel intravenös).

Hydrogele zur kontrollierten Feisetzung eines Wirkstoffes sind u. a aus US 7,291,673, US 2008/0014149 A1 und Yang J et al. 2010 (Macromol. Biosci., 10, 445-454) bekannt.

Die PEGylierung von pharmazeutischen Wirkstoffen wird insbesondere verwendet, um einerseits eine kontrollierte Freisetzung über einen gewünschten Zeitraum (Retardeffekt) zu erreichen und anderseits die Ausscheidung des Wirkstoffes über die Niere zu verzögern.

Zur PEGylierung von Polypeptiden sei u. a. auf US 4,179,337, sowie die Übersichtsartikel Kodera, M et al. 1998 (Prog Polym Sci. 23: 1233-71) und Veronese FM, Harris JM 2002 (Adv Drug Deliv Rev 54: 453-456) verwiesen.

Da eine irreversible Anbindung von PEG die pharmakologische Wirkung des Wirkstoffes beeinträchtigt, existiert eine Vielzahl von bekannten Ansätzen zur reversiblen PEGylierung.

Veronese FM (2001. Biomaterials 22: 405-17) beschreibt in einem allgemeinen Übersichtsartikel die reversible PEGylierung u. a. emzymkatalysiert an Glutamin-Seitenketten durch das Enzym Transglutaminase. Auch in einem Übersichtsartikel von Roberts MJ et al. 2002 (Adv Drug Deliv Rev 54: 459-476) werden mehrere Techniken für eine reversible PEGylierung von Peptiden und Proteinen erwähnt, insbesondere hydrolysierbare Esterbrücken, reduzierbare Disulfidbrücken.

EP1897561A, WO9930727, US 6,180,095, US6,720,306, WO0243663 beschreiben die Verwendung von Linkern, der durch eine 1,4- bzw. 1,6-Benzyleliminierung gespalten wird. In mehreren Publikationen wird dieser Linker in einem so genannten Doppel-Prodrug-Approach mit einer enzymatisch abspaltbaren Gruppe oder einer hydrolysierbaren Estergruppe kombiniert (Greenwald RB et al. 1999. J. Med. Chem. 42: 3657-3667; Lee S et al. 2001 Bioconjug Chem 12, 163-169; Greenwald RB et al. 2003. Bioconjug Chem. 14(2): 395-403).

Ein anderer Ansatz zur reversiblen PEGylierung ist die Trimethly-blockierte Laktonisierung (engl. trimethyl lock lactonization - TML), welche u. a. in US 5,965,119 und US 6,303,569 offenbart wird.

US 7,585,837 beschreibt einen Ansatz zur reversiblen PEGylierung durch Derivatisierung von funktionellen Gruppen mit 9-Fluorenylmethoxycarbonyl (Fmoc) oder 2-Sulfo-9-fluorenylmethoxycarbonyl (FMS), die leicht durch Basen abgespalten werden.

Guiotto et al. 2004 (J. Med. Chem. 47: 1280-9) beschreibt die Synthese, Charakterisierung und erste *in vivo* Tests von PEG-Konjugaten des Antitumormittels 10-Amino-7-ethylcamptothecin.

Eine Protease-basierte Prodrugstrategie basiert auf Peptidlinkern, die an klassische chemische Wirkstoffe ("small molecules") gebunden werden und Schnittstellen für Proteasen enthalten. Die zur Anwendung kommenden Proteasen sind hauptsächlich gewebespezifische Proteasen bzw. Proteasen, die bei der Gewebeumbildung (tissue remodeling) eine Rolle spielen (wie z. B. Cathepsin B, PSA (prostate specific antigen) und Matrixmetalloproteasen (MMP) aber auch die Serumproteasen Plasmin und Urokinase. Anwendungsgebiet der Wirkstoffe sind im Wesentlichen die Krebstherapie. Die Proteaseschnittstelle dient dem Tumortargeting. Hierzu sei auf den Übersichtartikel von Law B, Tung CH. 2009 (Bioconjug Chem. 20(9):1683-95) verwiesen. In der US 2004192769 wird der Ansatz erwähnt das Drugdelivery-System so zu gestalten, dass der Peptidlinker nicht von Serumproteasen, sondern erst nach Aufnahme in die Zielzelle abgespalten wird.

Li H et al. A 2010 (Angew. Chem. Int. Ed.. 49, 4930 -4933) wenden eine Protease-basierte ProdrugStrategie auf Peptidwirkstoffe an. Therapeutisch wirksame Peptide werden über den Peptidlinker mit einer Albumin-bindungstelle (albumin binding domain, ABD) gebunden. Im Blut bindet dieses Fusionspeptid zunächst an das Serumprotein Albumin und wird dann durch die Protease Thrombin (oder humaner Faktor Xa) abgespalten.

Nach wie vor besteht ein Bedarf an neuen Antibiotika.

Wünschenswerte Eigenschaften für Peptidantibiotika sind:
(i) eine gesteigerte Halbwertszeit in Säugetierserum durch eine höhere Proteaseresistenz und
(ii) unveränderte oder bevorzugt eine gesteigerte antimikrobielle Aktivität gegen einen oder mehrere Bakterienstämme, besonders humane Pathogene, oder Pilze oder andere mikrobielle Infektionen,
(iii) eine verminderte antigene Wirkung und dadurch reduzierte Immunreaktion und
(iv) die Peptide sind nicht toxisch gegenüber humanen Zellen, einschließlich Erythrozyten.

Die Wirkung Prolin-reicher antimikrobielle Peptide ist sehr komplex, da sie die Zellmembran durchdringen und in das Zytoplasma eindringen müssen, um ein spezielles intrazelluläres bakterielles Zielmolekül zu inhibieren, ohne jedoch auf Säugetierzellen und Blutzellen toxisch zu wirken. Ein anderer wichtiger Punkt ist die Stabilität der Peptide oder Peptidderivate gegenüber dem Abbau durch Peptidasen oder Proteasen in Blut und den Bakterien. Daher hat das ideale Peptid eine hohe antibakterielle Aktivität (kleine MHK-Werte), keine Zelltoxizität, keine hämolytische Aktivität und eine Halbwertszeit von mehreren Stunden in Blut.

Eine Aufgabe der Erfindung ist es neue Peptidantibiotika bereitzustellen, vorzugsweise mit gesteigerter Stabilität, verminderter Immunreaktion und verbesserter Pharmakokinetik.

In der Erfindung werden Peptidantibiotika reversibel über eine enyzmatisch spaltbare Peptidkette mit dem Polymer Polyethylenglykol (PEG) geschützt, um den Abbau des Wirkstoffs in Serum durch Proteasen und die Toxizität des Wirkstoffes zu reduzieren, sowie eine längere Verweildauer im Organismus zu erreichen (verbesserte Pharmakokinetik). Aus dieser chemisch definierten Verbindung werden die Wirkstoffe durch die Proteasen des Wirts (z.B. Patient, insbesondere Serumproteasen) oder der Bakterien freigesetzt. Die erfindungsgemäße Wahl des Polymers (Struktur, Länge) und des Peptidlinkers (Länge, Sequenz) ermöglichen die Kinetik der Wirkstofffreisetzung dem Wirkmechanismus des Wirkstoffs und dessen Metabolisierung oder Ausscheidung anzupassen. So kann durch eine geschickte Wahl der Struktur (Polymer, Linker) eine konstante Konzentration des Wirkstoffs während einer längeren Behandlungsdauer erreicht werden.

Erfindungsgemäß wird die Aufgabe in einem ersten Aspekt gelöst durch ein modifiziertes Peptid, ein Apidaecinderivat, welches eine Sequenz gemäß der allgemeinen Formel 1, 2 oder 3 aufweist:

NT-X₁X₂NX₃PVYIPX₄X₅RPPHP-CT (Formel 1)

NT-X₁NX₂X₃PVYIPX₄X₅RPPHP-CT (Formel 2)

NT-X₂NNX₃PVYIPX₄X₅RPPHP-CT (Formel 3)

wobei X₁ ein Aminosäurerest ist, dessen Seitenkette unter physiologischen Bedingungen positiv geladen ist, bevorzugt O (Ornithin),
wobei X₂ ein Aminosäurerest mit einer Aminogruppe in der Seitenkette ist,
wobei X₃ ein Aminosäurerest ist, dessen Seitenkette unter physiologischen Bendingungen positiv geladen ist, bevorzugt R (Arginin),
wobei X₄ ein Aminosäurerest ist, dessen Seitenkette unter physiologischen Bendingungen positiv geladen ist, bevorzugt R,
wobei X₅ Prolin oder ein Prolinderivat ist,
wobei CT der C-Terminus ist oder ein Peptid mit 1 bis 4 Aminosäureresten, bevorzugt ist ein Dipetid mit der Sequenz RL (Arg-Leu)
wobei NT der N-Terminus ist, der bevorzugt guanidiert ist.

Die restlichen Aminosäurereste haben jeweils die Bedeutung gemäß dem IUPAC-Einbuchstabencode.

Modifizierte Peptide gemäß Formel 1 und 2 sind besonders bevorzugt.

Das erfindungsgemäß modifizierte Peptid kennzeichnet sich dadurch aus dass an die Aminogruppe in der Seitenkette x₁ oder X₂ über einen Peptidlinker eine lineare oder verzweigte Polyethylenglykol-Polymerkette gebunden ist und der Peptidlinker 3 bis 10 Aminosäurereste lang ist und mindestens ein Arginin oder Lysin enthält.

Bevorzugt ist X₂ ein L-Ornithin.

Der Peptidlinker hat folgende Formel:

G(L)ₖRSG

mit L ausgewählt aus Alanin, Glycin und Serin und k ausgewählt ist aus ganzen Zahlen von 1 bis 6, bevorzugt 1, 2, 3 oder 4.

Die Polyethylenglykol-Polymerkette weist ein Molekulargewicht von 500 bis 40000 Da auf und ist an die alpha-Aminogruppe des ersten Aminosäurerests des Linkers gebunden.

Besonders bevorzugte Linker sind ausgewählt aus den Peptiden GRSG, GARSG, GAARSG, GAAARSG und GAAAARSG (SEQ ID No. 61 bis 65). Die Polyethylenglykol-Polymerkette ist bevorzugt an die alpha-Aminogruppe von Glycin als ersten Aminosäurerest des Linkers gebunden. Die Carboxylgruppe des letzten Aminosäurerest des Linkers ist an die Aminogruppe in der Seitenkette von X₂ (bevorzugt die delta-Aminogruppe eines L-Ornithinrest) gebunden. Trypsin und verwandte Serumproteasen schneiden jeweils nach dem R (oder K) im Peptidlinker, so dass das Peptid von der Polyethylenglykol-Polymerkette freigesetzt wird. Das freigesetzte Peptid enthält noch die Aminosäurenreste des Linkers, die ursprünglich rechts (C-terminal) des R (oder K) lagen, bevorzugt sind dies die Aminosäurenreste SG. Diese im freigesetzten Peptid verbleibenden Reste des Linkers sind weiterhin an die Aminogruppe in der Seitenkette von X₂ (bevorzugt die delta-Aminogruppe eines L-Ornithinrest) gebunden.

Erstaunlicherweise beinträchtigen diese verbleibenden Reste des Linkers nicht oder nur kaum die Aktivität des antimikrobiellen Peptids (Apidaecinderivat).

Besonders bevorzugte modifizierte Peptide (Apidaecinderivate) weisen folgende Peptidsequenzen auf:
ONORPVYIPRPRPPHPRL (SEQ ID No. 9) oder
OONRPVYIPRPRPPHPRL (SEQ ID No. 10) mit O = L-Ornithin
wobei die alpha-Aminogruppe des ersten Ornithins guanidiert ist und an die delta-Aminogruppe des zweiten Ornithins über einen wie oben ausgewählten Peptidlinker, bevorzugt ausgewählt aus **GRSG, GARSG** und **GAARSG** (SEQ ID No. 61 bis 63), eine lineare oder verzweigte Polyethylenglykol-Polymerkette gebunden ist.

Vorversuche zeigten, dass eine Modifikation des C-Terminus zu einem großen Aktivititätsverlust führt. Ein gewisser Aktivitätsverlust wird bei Einbau der Seitenkette an Position 1 (Formel 3, Orn-1 in SEQ ID No. 11) beobachtet, daher sind die Substitution von Asn-2 und Asn-3 gegen Orn (SEQ ID No. 9 und 10) besonders bevorzugt.

Etwas weniger bevorzugt modifizierte Peptide (Apidaecinderivate) weisen folgende Peptidsequenz auf:
ONNRPVYIPRPRPPHPRL (SEQ ID No. 11) mit O = L-Ornithin wobei die alpha-Aminogruppe des ersten Ornithins guanidiert ist und an die delta-Aminogruppe desselben Ornithins über einen Peptidlinker ausgewählt aus **GRSG, GARSG** und **GAARSG** (SEQ ID No. 61 bis 63) eine lineare oder verzweigte Polyethylenglykol-Polymerkette gebunden ist.

Bevorzugte modifizierte Peptide haben folgende Strukturen: wobei Linker für den Peptidlinker steht, der wie oben ausgewählt ist, besonders bevorzugt GRSG.

Ein weiterer Gegenstand der Erfindung ist ein modifiziertes Peptid, abgeleitet von Oncopeltus antibakterielles Peptid 4, welches eine Sequenz gemäß einer der allgemeinen Formeln 4 bis 6 aufweist:

NT-X₁-D₂-K₃-P₄-P₅-Y₆-L₇-P₈-R₉-P₁₀-X₂-P₁₂-P₁₃-R₁₄-X₃-I₁₆-Y₁₇-N₁₈-X₄-CT (Formel 4)

NT-X₁-D₂-K₃-P₄-P₅-Y₆-L₇-P₈-R₉-P₁₀-X₂-P₁₂-P₁₃-R₁₄-X₃-I₁₆-Y₁₇-N₁₈-N₁₉-X₄-CT (Formel 5)

NT-X₁-D₂-K₃-P₄-P₅-Y₆-L₇-P₈-R₉-P₁₀-X₂-P₁₂-P₁₃-R₁₄-X₃-I₁₆-P₁₇-N₁₈-X₄-CT (Formel 6)

X₁ ist ein Rest mit einer unpolaren, hydrophoben Seitenkette oder ein Aminosäurerest, dessen Seitenkette unter physiologischen Bendingungen positiv geladen ist, mit einer positiven Nettoladung oder einer unter physiologischen Bedingungen positiv geladenen Seitenkette;
D₂ ist ein Asparaginsäure- oder Glutaminsäurerest,
K₃ ist ein Rest mit einer positiven Nettoladung oder einer unter physiologischen Bedingungen positiv geladenen Seitenkette, bevorzugt Lysin oder Arginin,
X₂ und X₄ sind unabhängig von einander ausgewählt aus Resten mit einer positiven Nettoladung oder einer unter physiologischen Bedingungen positiv geladenen Seitenkette;
X₃ ist ein Rest mit einer positiven Nettoladung oder einer unter physiologischen Bedingungen positiv geladenen Seitenkette oder Prolin oder ein Prolinderivat;
L₇ und I₁₆ sind unabhängig von einander ausgewählt aus Resten mit einer unpolaren, hydrophoben Seitenkette, bevorzugt Leucin, Isoleucin, Valin und tert.-Leucin,
Y₆ und Y₁₇ sind jeweils Tyrosin, R₉ und R₁₄ sind jeweils Arginin, N₁₈ und N₁₉ sind jeweils Asparagin oder Glutamin, P₄, P₅, P₈, P₁₀, P₁₂, P₁₃ und P₁₇ sind unabhängig von einander ausgewählt aus Prolin und Prolinderivaten oder Hydroxyprolin und Hydroxyprolinderivaten,
wobei gegebenenfalls P₁₃ und R₁₄ vertauscht sind, und/oder
gegebenenfalls ein oder zwei der Reste ausgewählt aus D₂, P₄, P₅, P₈, P₁₀, P₁₂ P₁₃, P₁₇ und Y₁₇ durch einen beliebigen Rest ersetzt sind,
NT ist der N-Terminus der Aminosäure X₁,
CT ist die freie C-terminale Carboxylgruppe der C-terminalen Aminosäure (-COOH) oder eine modifizierte C-terminale Carboxylgruppe.

Dieses Peptid (Oncopeltus-Peptiderivat) zeichnet sich dadurch aus, dass an NT über einen Peptidlinker eine lineare oder verzweigte Polyethylenglykol-Polymerkette gebunden ist, wobei in den Peptidlinker eine tryptische Schnittstelle unmittelabr zwischen Polymer und dem antimikrobiellen Peptid eingefügt ist und der Linker folgende Formel:

G(L)ₖR

mit L ausgewählt aus Alanin, Glycin und Serin und k ausgewählt aus 1, 2 oder 3.

Die Polyethylenglykol-Polymerkette weist ein Molekulargewicht von 500 bis 40000 Da auf und ist an den Peptidlinker entweder direkt oder mittels eines kurzen organischen Linkers und/oder durch Ausbildung einer Thioetherbindung gekoppelt.

Besonders bevorzugte Linker sind ausgewählt aus den Peptiden GR, GAR und GAAR (SEQ ID No. 66).

X₅ und X₆ sind optional zusätzliche Reste. In dem Fall, dass X₅ und X₆ abwesend sind, hat das letzte Arginin (Arg) in der oben erwähnten Sequenz eine freie C-terminale Carboxylgruppe oder ist mit CT verbunden.

In dem Fall, in dem mindestens ein Rest X₅ und X₆ vorhanden ist, hat das Peptid beispielsweise eine Sequenz gemäß einer der allgemeinen Formeln 7 bis 9:

NT-X₁-D-K-P-P-Y-L-P-R-P-X₂-P-P-R-X₃-I-Y-N-X₄-X₅-X₆-COR₃ (Formel 7)

NT-X₁-D-K-P-P-Y-L-P-R-P-X₂-P-P-R-X₃-I-Y-N-X₄-X₅-COR₃ (Formel 8)

NT-X₁-D-K-P-P-Y-L-P-R-P-X₂-P-P-R-X₃-I-Y-N-X₄-X₆-COR₃ (Formel 9)

X₅ ist aus Prolin, Prolinderivaten oder einem neutralen Rest mit einer polaren Seitenkette (wie Asparagin, Glutamin) ausgewählt. Bevorzugte Reste X₅ sind aus den Gruppen ausgewählt, die Prolin, cis-4-Hydroxyprolin, trans-4-Hydroxyprolin, cis-3-Hydroxyprolin, trans-3-Hydroxyprolin, β-Cyclohexylalanin, 3,4-cis-Methanoprolin, 3,4-Dehydroprolin, Homoprolin, Pseudoprolin ebenso wie Asparagin, Glutamin, Citrullin, N-Methylserin, N-Methylglycin, Dihydroxyphenylalanin, N-Ethylasparagin, N-Ethylglycin, Homoserin, Penicillamin, Tetrahydropyranylglycin, allo-Threonin und 3,5-Dinitrotyrosin enthalten.

X₆ ist aus Prolin, Prolinderivaten, einem polaren Rest (wie Serin) oder einen hydrophoben Rest ausgewählt. Bevorzugte Reste X₆ sind aus den Gruppen ausgewählt, die Prolin, cis-4-Hydroxyprolin, trans-4-Hydroxyprolin, cis-3-Hydroxyprolin, trans-3-Hydroxyprolin, β-Cyclohexylalanin, 3,4-cis-Methanoprolin, 3,4-Dehydroprolin, Homoprolin oder Pseudoprolin, Serin, Threonin, δ-Hydroxylysin, Citrullin, Homoserin oder allo-Threonin ebenso Phenylalanin, N-Methylleucin, Leucin, Isoleucin, Valin, Methionin, *tert*.-Butylglycin, Cyclohexylalanin, Alanin, β-Alanin, 1-Aminocylcohexylcarbonsäure, N-Methylisoleucin, Norleucin, Norvalin, N-Methylvalin enthält oder es ist eine kurze Peptidsequenz mit vorzugsweise einem bis drei Resten, die bevorzugt ausgewählt sind aus Prolin, Isoleucin oder einem der zuvor erwähnten Reste.

Alternativ ist X₆ ein verzweigter Linker, der mehrere Peptideinheiten enthält. Dieser wird durch den Rest einer Aminosäure, die mehrere Aminogruppen enthält, wie z. B. Lysin, Hydroxylysin, Ornithin, 2,4-Diaminobuttersäure, 2,3-Diaminopropionsäure, 2,2'-Diaminopimelinsäure, Desmosin, Isodesmosin gebildet.

Die C-terminale Aminosäure ist zum Beispiel X₄ in der Formel 1, X₅ (in Formel 3) oder X₆ (in Formel 2 und 4).

Besonders bevorzugte modifizierte Peptide (Oncopeltus-Peptiderivate) weisen folgende Peptidsequenzen auf:
VDKPPYLPRPRPPROIYNO-NH₂ (SEQ ID No. 12) oder
VDKPPYLPRPRPHypRHypTleYNO-NH₂ (SEQ ID No. 13)
   mit O = L-Ornithin,
   Hyp = L-4-Hydroxyprolin und
   Tle = L-tertiär-Leucin (L-tertiär-Butylglycin),
wobei an die N-terminale Aminogruppe (alpha-Aminogruppe des ersten Aminorests V) über den Peptidlinker, bevorzugt GAR, eine lineare oder verzweigte Polyethylenglykol-Polymerkette gebunden ist. Der C-Terminus (CT) ist hier ein Carbonsäureamid (d. h. die Carboxylgruppe des letzten Orinithins ist zum Amid umgesetzt).

Die nachfolgend Ausführungen und erwähnten Vorzugsvarianten gelten allgemein für alle erfindungsgemäß modifizierten Peptide (Apidaecinderivate und Oncopeltus-Peptiderivate):
Die erfindungsgemäßen modifizierten Peptide enthalten bevorzugt mindestens 18 Aminosäurereste, bevorzugt bis zu 50 Aminosäurereste.

Für alle erfindungsgemäß modifizierten Peptide (Apidaecinderivate und Oncopeltus-Peptiderivate) gilt, dass die Polyethylenglykol-Polymerkette ein Molekulargewicht von 500 bis 40000 Da, besonders bevorzugt mindestens 5000 Da, aufweist. Die Polyethylenglykol-Polymerkette ist jeweils kovalent an den Peptidlinker gebunden, bevorzugt an die die N-terminale-Aminogruppe des Linkers, besonders bevorzugt die alpha-Aminogruppe des Glycins. Die Kopplung an den Peptidlinker erfolgt entweder direkt (z. B. mit einem NHS-Ester aktivierten Polyethylenglykol an die Aminogruppe) oder mittels eines kurzen organischen Linkers (bevorzugt C1 bis C10) und/oder durch Ausbildung einer Thioetherbindung (z. B. durch Derivatisierung der N-terminalen-Aminogruppe des Linkers, besonders bevorzugt die alpha-Aminogruppe des Glycins mit Iodessigsäure und Reaktion mit einem Thiol- modifizierten Polyethylenglykol). Die Polyethylenglykol-Polymerkette ist bevorzugt linear.

In allen erfindungsgemäßen Peptiden ist die tryptische Schnittstelle unmittelbar zwischen dem Polymer und dem antimikrobiellen Peptid eingefügt. Eine zusätzliche spaltbare Gruppe, die z. B. durch 1,6-Eliminierung entfernt wird, ist nicht enthalten.

Reste mit einer unter physiologischen Bedingungen positiv geladenen Seitenkette sind bevorzugt ausgewählt aus Arginin, Lysin, δ-Hydroxylysin, Homoarginin, 2,4-Diaminobuttersäure, β-Homoarginin, D-Arginin, Arginal (-COOH in Arginin ist ersetzt durch -CHO), 2-Amino-3-guanidinopropionsäure, Nitroarginin (bevorzugt N(G)-Nitroarginin), Nitrosoarginine (bevorzugt N(G)-Nitrosoarginin), Methylarginin (bevorzugt N-Methyl-Arginin), ε-N-Methyllysin, allo-Hydroxylysin, 2,3-Diaminopropionsäure, 2,2'-Diaminopimelinsäure, Ornithin, sym-Dimethylarginin, asym-Dimethylarginin, 2,6-Diaminohexinsäure, p-Aminobenzoesäure und 3-Aminotyrosin und weniger bevorzugt Histidin, 1-Methylhistidin und 3-Methylhistidin. X₁, X₂ und X₃ sind bevorzugt unabhängig voneinander aus dieser Liste ausgewählt.

Der Begriff Prolinderivat steht für einen von Prolin abgeleiteten Aminosäurenrest, der aus Prolin bevorzugt durch strukturelle Veränderung einer funktionellen Gruppe erhalten wird. Bevorzugte Prolinderivate sind ausgewählt aus, β-Cyclohexylalanin, 3,4-cis-Methanoprolin, 3,4-Dehydroprolin, Homoprolin oder Pseudoprolin. Der Begriff Hydroxyprolin schließt unter anderem cis-4-Hydroxyprolin, trans-4-Hydroxyprolin, cis-3-Hydroxyprolin und trans-3-Hydroxyprolin mit ein. Der Begriff Hydroxyprolinderivat steht entsprechend für einen von Hydroxyprolin abgeleiteten Aminosäurenrest, der aus Hydroxyprolin bevorzugt durch strukturelle Veränderung einer funktionellen Gruppe erhalten wird. Bevorzugte Hydroxyprolinderivate sind ausgewählt aus Hydroxy-β-Cyclohexylalanin und den oben genannten Prolinderivaten, die mit einer Hydroxylgruppe substituiert sind.

Ein neutraler Rest ist ein Rest mit einer unter physiologischen Bedingungen ungeladenen Seitenkette.

Ein polarer Rest weist bevorzugt mindestens eine polare Gruppe in der Seitenkette auf. Diese sind bevorzugt ausgewählt aus Hydroxyl-, Sulfhydryl-, Amin-, Amid- oder Estergruppen oder anderen Gruppen, welche die Ausbildung von Wasserstoffbrücken erlauben.
Bevorzugte neutrale polare Reste sind ausgewählt aus Asparagin, Cystein, Glutamin, Serin, Threonin, Tyrosin, Citrullin, N-Methylserin, Homoserin, allo-Threonin und 3,5-Dinitrotyrosin und β-Homoserin,

Die Reste mit einer unpolaren, hydrophoben Seitenkette sind unter physiologischen Bedingungen ungeladene Reste, bevorzugt mit einem Hydropathie-Index über 0, besonders bevorzugt über 3. Bevorzugte unpolare, hydrophoben Seitenketten sind ausgewählt aus Alkyl-, Alkylen- Alkoxy-, Alkenoxy-, Alkylsulfanyl- und Alkenylsulfanylresten mit 1 bis 10, bevorzugt 2 bis 6 C-Atomen, oder Arylresten mit 5 bis 12 C-Atomen. Bevorzugte Reste mit einer unpolaren, hydrophoben Seitenkette sind ausgewählt aus Leucin, Isoleucin, Valin, Methionin, Alanin, Phenylalanin, *N*-Methylleucin, *tert.-*Butylglycin, Cyclohexylalanin, β-Alanin, 1-Aminocylcohexylcarbonsäure, *N*-Methylisoleucin, Norleucin, Norvalin und *N*-Methylvalin.

Unter physiologischen Bedingungen sind ein pH-Wert von pH 6 bis 8 und eine Temperatur von 30°C bis 40°C zu verstehen, bevorzugt eine Temperatur von 37°C, ein pH-Wert von 7,4 und ein osmotischer Druck von 300 mosmol/kg.

NT ist der freie N-Terminus von X₁ oder eine modifzierte N-terminale Aminogruppe. CT ist die freie C-terminale Carboxylgruppe der C-terminalen Aminosäure (-COOH) oder eine modifizierte C-terminale Carboxylgruppe. "Modifizierte N-terminale Aminogruppe" und "modifizierte C-terminale Carboxylgruppe" bedeutet, dass die Aminogruppe bzw. Carboxylgruppe verändert ist (z. B. reduziert oder substituiert).

NT stellt somit den freien N-Terminus der Aminosäure X₁ oder eine Modifikation der N-terminalen Aminogruppe (welche die N-terminale Aminogruppe der Aminosäure X₁ durch NT ersetzt) mit der generellen Formel NR₁R₂, dar. NT = NR₁R₂, wobei R₁ und R₂ unabhängig voneinander sind und bevorzugt aus Wasserstoff oder aus folgenden Gruppen ausgewählt werden:
(i) einer geradkettigen, verzweigten, zyklischen oder heterozyklischen Alkylgruppe, wie z. B. Methyl, Ethyl, n-Propyl, Isopropyl, n-Butyl, Isobutyl oder Cyclohexyl;
(ii) einer geradkettigen, verzweigten, zyklischen oder heterozyklischen Alkanoylgruppe, wie z. B. Acetyl oder Methanoyl (Formyl), Propionyl, n-Butyryl, Isobutyryl, Pentanoyl, Hexanoyl oder Cyclohexanoyl;
(iii) eine Reportergruppe, bevorzugt ein Fluoreszenzfarbstoff (wie z. B. Fluorescein, Alexa488) oder Biotin;
(iv) zusammen mit COR₃ (siehe unten) einen Linker zwischen N- und C-Terminus um ein zyklisches Peptid zu erhalten, z. B. basierend auf Guanidin, Ethyleneglycololigomere, 2,4-Diaminobuttersäure, 2,3-Diaminopropionsäure, 2,2'-Diaminopimelinsäure, Desmosin oder Isodesmosine.
(v) einen Linker zur Kopplung eines weiteren Peptids oder Peptidderivats (Y₁) über eine spezifische chemische oder enzymatische Reaktion, z.B. basierend auf Iod-, Brom- oder Chloralkansäuren (z.B. Iodessigsäure) oder Maleimid zur Kopplung an ein Thiol-haltiges Peptid oder auch einer anderen reaktiven Gruppe (z.B. Aminogruppe, Thiolgruppe) zur Kopplung eines zweiten Peptids oder Peptidderivats (z.B. als Aktivester, Aldehyd oder Thioester) als Träger- oder Carrierprotein.
(vi) einen wie in (v) genannten Linker, an den ein weiteres Peptid oder Peptidderivat Y₁ gekoppelt ist.

Beispiele für N-terminale Modifikationen sind acetylierte, formylierte und bevorzugt guanidierte N-Termini.

Bevorzugt ist über NT ein weiteres Peptid oder Peptidderivat Y₁ gekoppelt. Y₁ ist vorzugsweise ein Biopolymer (z.B. Peptid), welches das antimikrobielle Peptid nach Formel 1 in Bakterien einschleust und damit die Aktivität des antimikrobiellen Peptids gegenüber diesem Bakterium erhöht und/oder in Säugerzellen einschleust und damit die Behandlung von Bakterien ermöglicht, die sich in Säugerzellen verstecken. Y₁ ist über NT entweder permanent (z.B. Peptid- oder Amidbindung für NT=NH₂ oder Thioether für NT=SH, Iodacetat oder Maleimid) oder durch eine Verbindung, die unter bestimmten Bedingungen spaltbar ist (wie z. B. Disulfidbrücken oder säurelabile Linker), mit X₁ des Peptids verknüpft. Bevorzugte Sequenzen für Y₁ sind Zell-penetrierende Peptide (CPP, cell penetrating peptides), beispielsweise Penetratin, Tat-Peptide, amphipathische Modell-Peptide (model amphipathic peptides) und Transportans (Langel, U. in Handbook of Cell-Penetrating Peptides 5-28. CRC - Taylor & Francis Group, 2006).

Ein Linker ist eine Bezeichnung für Moleküle oder Molekülgruppen, die zum Verknüpfen von zwei Substanzen herangezogen werden, bevorzugte Linker enthalten zwei reaktive Gruppen (wie z. B. Iodacetat, Maleimid, Imido- oder NHS-Ester oder Hydrazid), die durch eine Molekülbrücke (z. B. Polyethylenglykol) mit bevorzugt 10 bis 20 C-Atomen verbunden sind.

CT ist die freie C-terminale Carboxylgruppe der C-terminalen Aminosäure (-COOH) oder eine modifizierte C-terminalen Carboxylgruppe, vorzugsweise mit der allgemeinen Formel COR₃ (R₃ ersetzt die Hydroxylgruppe der letzten Aminosäure), X₅-COR₃ oder X₆-COR₃ oder X₅X₆-COR₃.

COR₃ ist vorzugsweise aus der folgenden Gruppe ausgewählt:
i. Carboxyl (R₃ ist eine freie Hydroxylgruppe), ein Ester (R₃ ist eine Alkoxygruppe), ein Amid (R₃ ist ein Amin) oder ein Imid;
ii. ein Linker, der zusammen mit NT, die N- und C-Termini zu einem zyklischen Peptid verbrückt;
iii. COR₃, worin R₃ entweder ein zusätzlicher Aminosäurerest ist, der aus der Gruppe die Pro, Ile, Leu, Arg und Gln enthält, ausgewählt ist, oder worin R₃ ein Peptid, mit bevorzugt zwei bis sechs Aminosäuren ist, wovon mindestens eine Aminosäure aus der Gruppe, die Pro, Ile, Leu, Arg oder Gln enthält, ausgewählt ist, wobei diese mit einem Mitglied aus der Gruppe mit Carboxyl (R₃ ist eine freie Hydroxylgruppe), einem Ester (R₃ ist ein Alkohol, wie Methanol, Ethanol, Propanol, iso-Propanol oder Butanol), einem Amid (R₃ ist ein Amid) oder ein Imid (R₃ ist ein Alkylamin oder Dialkylamin, wie Methylamin, Ethylamin, Dimethylamin oder Zyklohexylamin) substituiert ist.
iv. COR₃ worin R₃ eine zusätzliche, verzweigte Aminosäure ist, um eine Dimer- oder Oligomerstruktur auszubilden, wie beispielsweise Lysin, Hydroxylysin, Ornithin, 2,4-Diaminobuttersäure, 2,3-Diaminopropionsäure, 2,2'-Diaminopimelinsäure, Desmosin, Isodesmosin oder eine Kombination aus diesen verzweigten Aminosäuren.
v. ein Linker zur Kopplung eines weiteren Peptids oder Peptidderivats (Y₁) über eine spezifische chemische oder enzymatische Reaktion, z.B. basierend auf Iod-, Brom- oder Chloralkansäuren (z.B. Iodessigsäure) oder Maleimid zur Kopplung an ein Thiol-haltiges Peptid oder auch einer anderen reaktiven Gruppe (z.B. Aminogruppe, Thiolgruppe) zur Kopplung eines zweiten Peptids oder Peptidderivats (z.B. als Aktivester, Aldehyd oder Thioester) als Träger- oder Carrierprotein.
vi. einen wie in (v) genannten Linker, an den ein weiteres Peptid oder Peptidderivat Y₁ gekoppelt ist

Auf diesem Weg können C-terminale Peptidderivate als Ester (R₃ = Alkoxy), Amid (R₃ = Amin, z. B.-NH₂ oder Imin, z. B. -NHC₃H₇) oder Imid oder ein Peptid, das um weitere Aminosäuren verlängert wurde, die aus der Gruppe, die Pro, Ile, Arg und Val enthält, ausgewählt wurde und ebenfalls wieder am C-Terminus als Ester, Amid oder Imid modifiziert sind, erhalten werden. Weitere Peptidderivate können durch Modifikationen der N-terminalen oder C-terminalen Enden der Peptide gebildet werden. Diese Änderungen können beispielsweise eine zusätzliche Alkyl- oder Alkanoylgruppe (entweder mit einer geraden Kette oder verzweigt, zyklisch oder heterozyklisch) oder eine zusätzliche Guanidinogruppe oder ein zusätzliches Makromolekül oder ein Reporterrest sein, der entweder permanent oder durch eine Verbindung, die unter bestimmten Bedingungen spaltbar ist (wie Disulfidbrücken oder säurelabile Linker), verknüpft ist.
Bevorzugt erfolgt eine Modifikation des C-Terminus mittels Thioestersynthese und anschließender Substitition mit primären Aminen.

Alle natürlichen Aminosäuren, unnatürlichen Aminosäuren oder Aminosäurederivate (wie z.B. Iminosäuren), welche die erfindungsgemäßen Peptide oder Peptidderivate bilden, können entweder in der L- oder D-Konformation vorliegen. Wenn nicht anders spezifiziert sind die Bausteine in den Sequenzen jedoch bevorzugt in der L-Konformation.

Die Modifikationen der N- und C-Termini erlauben das Koppeln der Peptide an andere Gruppen, wie zum Beispiel andere Aminosäuresequenzen (dabei werden eventuell multimere Peptide oder Proteine geschaffen) oder andere Biomoleküle, welche die Funktion eines Carriers oder Labels haben, beispielsweise von Y₁ über NT. In einer speziellen Ausführungsform fungiert das Molekül als Träger um die bakterielle Infektion in Säugerzellen zu bekämpfen oder das antibakterielle Peptid und Peptidderivat in Bakterien zu transportieren, in die das antibakterielle Peptid nicht allein eindringen kann (z.B. Gram-positive Bakterien). Beispiele für solche Zell-penetrierenden Peptide (CPP) sind beispielsweise Penetratinen, Tat-Peptide, amphipathischen Modell-Peptiden (model amphipathic peptides) und Transportans. Zudem kann der Ort der Infektion durch die gekoppelte Struktur (Target-Molekül) erkannt werden und dadurch die antibiotische Substanz in die Nähe der (Bakterien-) Zelle gebracht werden, um diese bekämpfen. Solche Target-Moleküle sind z.B. Moleküle, die bekanntermaßen an Lipopolysaccharid (LPS)-Moleküle binden, welche die Außenseite der Gram-negativen Bakterien bilden. Bekannte Verbindungen für diese Anwendung sind beispielsweise Ankerpeptide, wie das AcmA-Motiv aus Lactobacillus oder ein gegen Lipopolysaccharid gerichteter Antikörper. Die letztere Variante ist bevorzugt, da sie auch einen intrinsischen antibiotischen Effekt hat und deshalb zur Steigerung der Aktivität der erfindungsgemäßen Peptide genutzt werden kann.

Durch die Ankopplung einer Zell-penetrierenden Peptidsequenz, wie Penetratin, kann einerseits die Aktivität gegenüber Gram-negativen und Gram-positiven Bakterien gesteigert bzw. das Wirkungsspektrum auf andere Gram-positive und Gram-negative Bakterien erweitert werden und andererseits die antimikrobiellen Peptide in Säugerzellen eingeschleust werden, so dass auch in diesen Zellen verborgene Bakterien, Pilze oder Viren erreicht werden können. Bestandteil der Erfindung ist die Kopplung des Penetratins über eine Thioetherbrücke. Dabei wurde der C-Terminus des Penetratins um ein Cystein verlängert und an das N-terminal mit Iodessigsäure markierte antimikrobielle Peptid gekoppelt.

Der Ausdruck "Peptid", wie hier verwendet, steht für eine Sequenz von Aminosäuren, die über eine Peptidbindung verknüpft sind, wobei die Aminosäuren bevorzugt aus den zwanzig proteinogenen Aminosäuren ausgewählt sind und worin die Aminosäuren in der L-Konfiguration oder D-Konfiguration, oder im Fall von Isoleucin und Threonin auch in der D-allo-Konfiguration vorliegen können (nur Inversion eines der beiden chiralen Zentren). Umfasst sind zudem Peptide, die durch Substitutionen und/oder Modifikationen von einem oder mehreren Aminosäureresten durch chemische Gruppen verändert wurden, wobei diese chemischen Gruppen andere als die natürlichen Protein-bildenden Aminosäurereste sind, wie z.B. nicht-proteinogene α-Aminosäuren, β-Aminosäuren oder Peptide mit verändertem Rückgrat. Der Begriff "verändertes Rückgrat" bedeutet, dass mindestens eine Peptidbindung chemisch modifziert ist, d. h. ersetzt ist durch eine unter physiologischen Bedingungen nicht spaltbare Bindung, die nicht durch Endoproteasen geschnitten werden kann.

Bevorzugt ist die nicht spaltbare Bindung eine modfizierte Peptidbindung wie z. B. eine reduzierte Peptidbindung, eine alkylierte Amidbindung oder eine Thioamidbindung. Eine reduzierte Amidbindung ist eine Peptidbindung in der die Carbonylgruppe (C=O) zu einer Hydroxylgruppe (HCOH) oder einer Methylengruppe (CH₂) reduziert ist. Eine alkylierte Amidbindung ist eine entweder am Stickstoff (N-alpha) oder Kohlenstoffatom (C-alpha) alkylierte Peptidbindung. Der Alkylrest hat bevorzugt 1 bis 3 C-Atome. Ein Beispiel ist die N-Methylierung.
Außerdem umfasst der Begriff verändertes Rückgrat andere Gruppen, die geeignet sind, eine kovalente Bindung sowohl mit der COOH-Gruppe des vorangegangenen Aminosäurerestes als auch der NH₂-Gruppe des folgenden Aminosäurerestes zu bilden, und die daher nicht notwendigerweise die Peptidrückgrat-Struktur aufrecht erhalten, wie z. B. Zuckeraminosäure-Dipeptid-Isostere, Azapeptide, 6-Homopolymere, gamma-Peptide, Depsipeptide (Esterbrücken im Rückgrat), Y-Lactam-Analoga, Oligo(phenylenethylen)e, vinyloge Sulfonpeptide, poly-N-substituierte Glycine oder Oligocarbamate. Modifikationen des Rückgrats sind an Positionen bevorzugt, die anfällig für enzymatischen Abbau sind, besonders an den sechs C-terminalen Resten der Peptide gemäß Formel 4 bis 9 (Oncocin- bzw. Oncopeltuspeptidderivate), besonders bevorzugt Positionen 14 bis 19, R-X₃-I₁₆-Y₁₇-N₁₈-X₄). Daher ist vorzugsweise mindestens eine der Bindungen zwischen X₃-I₁₆ (z.B. Arg-Ile), N₁₈-X₄ (z.B. Asn-Arg), X₄-NH₂ (z.B. Arg-NH₂), X₆-X₇ (z.B. Arg-Leu oder Arg-Ile) eine für Proteasen nicht spaltbare Bindung. Diese nicht spaltbare Bindung ist vorzugsweise aus der Gruppe der reduzierten Amidbindungen, alkylierten Amidbindungen oder Thioamidbindungen ausgewählt.

Die erfindungsgemäßen Peptide sind bevorzugt linear. Alternativ sind die Peptide, insbesondere die Apidaecinderivate, auch zyklisch, d. h. bevorzugt die erste (N-Terminus) und die letzte Aminosäure (C-Terminus) sind über eine Peptidbindung oder einen Linker verknüpft. Umfasst sind jedoch auch Zyklisierungen zwischen einer Seitenkette (z. B. Lysin) und C-Terminus, einer Seitenkette (z. B. Glutaminsäure oder Asparaginsäure) und N-Terminus oder zwischen zwei Seitenketten (z. B. Lysin und Glutaminsäure oder Asparaginsäure).

Weitere Bestandteile dieser Erfindung sind die Methoden zur Herstellung der oben erwähnten neuartigen antibiotisch wirksamen Verbindungen.

Die Peptide oder Peptidderivate dieser Erfindung können entweder synthetisch oder, wo anwendbar, rekombinant mit konventionellen Methoden hergestellt werden. Vorzugsweise werden die Peptide oder Peptidderivate dieser Erfindung konventionell mit den bekannten Synthesetechniken hergestellt, wie beispielsweise von Merrifield beschrieben. Alternativ werden die in dieser Erfindung beschriebenen Peptide durch rekombinante Techniken hergestellt, indem man ein DNA-Fragment, welches eine Nukleinsäuresequenz enthält, die für eines der oben beschriebenen Peptide kodiert, kloniert, und z. B. in einem Mikroorganismus oder einer Wirtszelle exprimiert. Die kodierenden Nukleinsäuresequenzen können synthetisch hergestellt werden oder durch seitenspezifische Mutagenese einer existierenden Nukleinsäuresequenz (z.B. Sequenz die das Wildtyp-Oncopeltus 4 kodiert) gewonnen werden. Die so hergestellte kodierende Sequenz kann von der RNA (oder DNA) mit entsprechend hergestellten Primern in einer Polymerasekettenreaktion (PCR) mit bekannten Techniken amplifiziert werden. Nach Reinigung, beispielsweise mittels Agarose-Gelelektrophorese, wird das PCR-Produkt in einen Vektor ligiert und letztlich die Wirtszelle mit dem entsprechenden rekombinanten Plasmid transformiert. Rekombinante Techniken sind für unterschiedliche Wirtszellen bekannt, beispielsweise *E. coli, Bacillus, Lactobacillus, Streptomyces,* Säugerzellen (z.B. CHO (Chinese hamster ovary) oder COS-1 Zellen), Hefezellen (z.B. *Saccharomyces, Schizophyllum*), Insektenzellen oder virale Expressionssysteme (z.B. Baculovirus System). Nach konventioneller rekombinanter Herstellung können die Peptide dieser Erfindung aus den Wirtszellen isoliert werden, entweder mit klassischen Zellaufschlusstechniken oder vom Zellmedium mit konventionellen Methoden, z.B. Flüssigchromatographie, insbesondere der Affinitätschromatographie. Das antimikrobielle Peptid kann als einzelnes Peptid oder als Oligomer exprimiert werden. Dabei können die Oligomere mehrere Peptidsequenzen enthalten, die über den N- oder C-Terminus verknüpft sind, oder gar einen N- oder C-terminalen Tag enthalten, der die einfachere Reinigung der rekombinanten Peptide oder Proteinkonstrukte erlaubt. Konventionelle molekularbiologische Techniken und seitenspezifische Mutagenese können eingesetzt werden, um die Sequenz weiter zu verändern und so die gewünschten nicht-nativen Peptidsequenzen zu erhalten. Diese rekombinaten Techniken wurden bereits für viele antimikrobielle Peptide einschließlich Apidaecin (s. z. B. Maeno M et al. 1993. Biosci Biotechnol Biochem57:1206-7) angewandt.

Es ist auch möglich nicht natürlich vorkommende Aminosäuren gentechnisch in die Peptide einzubringen (Noren C et al. 1989.Science 244: 182-8; Ellman J et al. 1991 Methods Enzymol. 202: 301-36).

Anschließend können die Peptide aus der Wirtszellkultur oder dem *in vitro-* Translationssystem isoliert werden. Dies kann mit den üblichen Techniken zur Proteinreinigung und -isolierung erreicht werden, die Stand der Technik sind. Solche Techniken können beispielsweise die Immunadsorption oder Affinitätschromatographie beinhalten. Es ist außerdem möglich, die Peptide während der Synthese mit einem Tag zu versehen (z.B. Histidin-Tag), der eine schnelle Bindung und Reinigung gestattet. Der Tag kann nachträglich enzymatisch abgespalten werden, um die aktive Peptidsequenz zu erhalten.

Falls das Peptid selbst nicht kodiert oder exprimiert werden kann, aber sehr ähnlich zu einem kodierbaren oder exprimierbaren Peptid ist, kann die Methode zunächst auf das ähnliche Peptid angewandt werden, um dieses nachträglich in einem oder mehreren Schritten chemisch oder enzymatisch in das gewünschte Peptid oder Peptidomimetika zu überführen.

Die erfindungsgemäßen modifizierten Peptide können einzeln, in Kombination, als Multimere oder als verzweigte Multimere eingesetzt werden. Sinnvolle Kombinationen der erfindungsgemäßen Peptide umfassen Konkatamere, in denen die erfindungsgemäßen Peptide seriell miteinander oder über Spacer miteinander verknüpft sind, z. B. in der Form eines Peptiddimers oder eines Peptidtrimers usw., indem die einzelnen Peptide aneinander gereiht sind. Dieses Multimer kann aus Peptiden oder Peptidderivaten mit identischen Sequenzen oder verschiedenen Sequenzen gemäß Formel 1 zusammengesetzt sein.

Die modifizierten Peptide können zusätzlich an ein biokompatibles Protein gekoppelt werden, beispielsweise humanes Serumalbumin, humanisierte Antikörper, Liposomen, Mizellen, synthetische Polymere, Nanopartikel und Phagen. Alternativ können Multimere, in denen die erfindungsgemäßen Peptide oder Peptidderivate individuell kombiniert sind, in der Form von Dendrimeren oder Clustern hergestellt werden, wobei drei oder mehr Peptide an ein Zentrum gebunden sind.

In einer Ausführungsform können mehrere Peptide als multimere Konstrukte oder Anordnung hergestellt werden. So können beispielsweise optional Aminosäuren (z.B. Gly-Ser-) oder andere Spacer basierend auf Aminosäuren oder anderen chemischen Verbindungen an den N- oder C-Terminus angehängt werden, um zwei oder mehr Peptide untereinander zu verknüpfen oder an einen Träger zu koppeln. Diese Anordnung kann die Form von einem oder mehreren der oben beschriebenen synthetischen Peptide gekoppelt an ein Trägerprotein annehmen. Alternativ enthält eine Anordnung mehrere Peptide, jedes als multiples antigenes Peptid exprimiert, optional an ein Trägerprotein gekoppelt. In einer weiteren Variante sind die ausgewählten Peptide sequentiell verknüpft und werden als rekombinantes Protein oder Polypeptid exprimiert. In einer Ausführungsform werden mehrere Peptide sequentiell, mit oder ohne Aminosäuren als Spacer dazwischen, verknüpft, um ein größeres rekombinantes Protein zu erhalten. Alternativ kann das rekombinante Protein an ein Trägerprotein fusioniert werden.

In einer anderen Ausführungsform enthalten die multimeren Konstrukte mindestens zwei Peptide, wobei ein Peptid über eine beliebige Aminosäure an die anderen Peptide gekoppelt wird. Eine beliebige Anzahl weiterer Peptide können an beliebige weitere Aminosäuren dieser Peptide angehängt werden. In einer weiteren Ausführungsform einer multimeren Anordnung, welches mindestens zwei Peptide enthält, ist das zweite oder sind die weiteren Peptide an ein verzweigtes Gerüst der anderen Peptide der Grundstruktur gekoppelt. Alternativ ist jedes weitere Peptid kovalent über die Gruppe NT oder CT an ein anderes Peptid der Anordnung verknüpft.

In einer anderen Ausführungsform eines multimeren Konstrukts oder einer Anordnung mit mindestens zwei Peptiden, sind mindestens eins oder mehrere Peptide an einen Träger gebunden. In einer anderen Ausführungsform sind ein oder mehrere der genannten Peptide ein synthetisches Peptid, das an ein Trägerprotein fusioniert ist. Weiterhin gibt es die Alternative mehrere der oben beschriebenen Peptide mit oder ohne flankierende Sequenzen sequentiell zu einem linearen Polypeptid zu kombinieren. Die Peptide oder das Polypeptid sind entweder an den gleichen Träger gekoppelt oder unterschiedliche Peptide können individuell als Peptide an eins oder unterschiedliche immunologisch inerte Trägerproteine gekoppelt werden.
Geeignete Träger können die Stabilität, die Darreichung oder die Produktion verbessern, oder das Aktivitätsspektrum der Peptide verändern. Beispiele für Träger sind humanes Albumin, Polyethylenglykol oder andere Biopolymere bzw. andere natürlich oder nicht-natürlich vorkommende Polymere. In einer Ausführungsform, ist die Hauptkomponente vorzugsweise ein Protein oder anderes Molekül, das die Peptidstabilität erhöhen kann. Eine erfahrene Person kann einfach eine geeignete Kopplungseinheit auswählen.

In noch einer anderen Ausführungsform sind die Peptide in der Form eines multiplen Antigenpeptides (multiple antigenic peptide; MAP) angeordnet. Dieses System nutzt eine zentrale Einheit aus Lysinresten, an die mehrere Kopien des gleichen erfindungsgemäßen Peptids synthetisiert werden. Jedes MAP enthält mehrere Kopien eines oder mehrerer der erfindungsgemäßen Peptide. Eine Ausführungsart eines MAP enthält mindestens drei, vorzugsweise aber vier oder mehr Peptide. Ein Fachmann kann einfach eine beliebige Anzahl multimerer Verbindungen gemäß den in obiger Formel identifizierten Peptiden herstellen. Alle derartigen multimeren Arrangements und Konstrukte sollen Bestandteil dieser Erfindung sein.

Weitere Kombinationen in der Form von Multimeren können an der Oberfläche von Partikeln hergestellt werden, wobei die Peptide oder Peptidomimetika an deren Oberfläche präsentiert werden. Der Partikel kann dann als Träger eines Peptids oder Peptidomimetikas fungieren und kann gleichzeitig als detektierbarer Marker wirken. Multimere können beispielsweise durch N-terminale Biotinylierung des N-terminalen Endes der Peptid- oder Peptidomimetika-Ketten und anschließende Komplexbildung mit Streptavidin erhalten werden. Da Streptavidin vier Biotinmoleküle oder -konjugate mit hoher Affinität binden kann, werden mit dieser Methode sehr stabile tetramere Peptidkomplexe erhalten. Multimere können aus identischen oder unterschiedlichen erfindungsgemäßen Peptiden oder Peptidomimetika hergestellt werden. Vorzugsweise enthalten die erfindungsgemäßen Multimere zwei oder mehr Peptid oder Peptidomimetika, in welcher jede Komponente einen gewissen Anteil zur bioziden Aktivität beiträgt (Zielerkennung, antimikrobielle Aktivität, Reinigung).

Ein anderer Gegenstand dieser Erfindung ist der Einsatz der erfindungsgemäßen Peptide oder Peptidderivate in der Medizin oder Pharmazie, z.B. zur Therapie mit einem Antibiotikum oder in einer Zusammensetzung mit antimikrobieller (insbesondere bakteriozider) Aktivität.

Gegenstand der Erfindung sind auch die erfindungsgemäß modifizierten Peptide zur Verwendung in der Medizin, als Antibiotikum, in einem Desinfektions- oder Reinigungsmittel, als Konservierungsmittel oder in einem Verpackungsmaterial. Besonders eigen sich die erfindungsgemäß modifizierten Peptid zur Behandlung von mikrobiellen, bakteriellen oder Pilz-Infektionen.

Gegenstand der Erfindung ist auch die Verwendung der erfindungsgemäß modifizierten Peptide zur Herstellung eines Arzneimittels, insbesondere eines Antibiotikums, insbesondere zur Behandlung von mikrobiellen Infektionen, z.B. durch Bakterien, Viren und Pilze.

Ein weiterer Gegenstand dieser Erfindung sind pharmazeutische Zusammensetzungen, die ein oder mehrere erfindungsgemäß modifizierte Peptide oder multimere Konstrukte unabhängig von der Anwesenheit anderer pharmazeutischer aktiver Verbindungen enthalten.

Bestandteil dieser Erfindung ist auch die Verwendung der erfindungsgemäßen Peptide als Pharmazeutikum und/oder zur Herstellung eines Wirkstoffs, der als Antibiotikum eingesetzt werden kann.

Die modifizierten Peptide können auch einzeln in pharmazeutischen Produkten eingesetzt werden. Alternativ können ein oder mehrere modfizierte Peptide, wie oben beschrieben, an eine andere Verbindung fusioniert oder konjugiert werden, um die Pharmakokinetik oder Bioverfügbarkeit zu steigern, ohne dass eine Immunantwort ausgelöst wird. Eine beliebige Anzahl einzelner Peptide oder multimerer Konstrukte können miteinander gemischt werden, um eine einzelne Zusammensetzung herzustellen.

Eine erfindungsgemäße pharmazeutische Zusammensetzung enthält eine therapeutisch wirksame Menge eines oder mehrerer modfizierter Peptide dieser Erfindung. Einmal zusammengestellt, kann die erfindungsgemäße pharmazeutische Zusammensetzung dem Subjekt direkt verabreicht werden, um mikrobielle (insbesondere bakterielle) Infektionen zu behandeln. Dazu wird dem zu behandelnden Subjekt eine therapeutisch wirksame Menge einer erfindungsgemäßen Zusammensetzung verabreicht.

Die erfindungsgemäßen Zusammensetzungen sind dazu bestimmt Infektionen eines mit Bakterien oder Pilzen infizierten Säugetiers einschließlich des Menschen zu behandeln. Mindestens ein oder alternativ auch mehrere erfindungsgemäße Peptide oder multimere Konstrukte können zu einer antimikrobiell (insbesondere antibakteriell oder fungizid) wirksamen Zusammensetzung mit einem pharmakologisch vertretbaren Träger oder anderen Komponenten gemischt werden. Für den Gebrauch einer derartigen Zusammensetzung wird das ausgewählte Peptid vorzugsweise synthetisch oder auch rekombinant, wie oben beschrieben, hergestellt.

Die direkte Verabreichung dieser Zusammensetzung erfolgt lokal oder systemisch, bevorzugt oral, parenteral, intraperitoneal, intravenös, intramuskulär, pulmonal oder interstitiell ins Gewebe.

Die pharmazeutische Zusammensetzung kann weiter geeignete und pharmazeutisch akzeptable Träger, Streckmittel oder Lösungsmittel enthalten und die Form einer Kapsel, Tablette, Pastille, Dragee, Pille, Tropfen, Zäpfchen, Puder, Spray, Impfstoff, Salbe, Paste, Creme, Inhalat, Pflaster, Aerosol oder ähnlichem aufweisen. Als pharmazeutisch akzeptable Trägerstoffe können Lösungsmittel, Streckmittel oder andere flüssige Bindemittel wie Dispersions- oder Suspensionshilfsmittel, oberflächenaktive Agenzien, isotonische Wirkstoffe, Dickungsmittel oder Emulgatoren, Konservierungsmittel, Umhüllungsmittel (encapsulating agent), feste Bindestoffe oder Gleitmittel eingesetzt werden, je nachdem was für die jeweilige Dosierung am Besten geeignet ist und zugleich mit dem Peptid, Peptidomimetika (Pepidderivat), Peptid-Konjugat oder Peptidmimetika-Konjugat kompatibel ist.

Die pharmazeutische Zusammensetzung enthält daher vorzugsweise einen pharmazeutisch akzeptablen Träger. Der Begriff "pharmazeutisch akzeptabler Träger" umfasst dabei auch einen Träger zur Verabreichung der therapeutischen Zusammensetzung, wie beispielsweise Antikörper oder Polypeptide, Gene oder andere therapeutische Mittel. Der Begriff bezieht sich auf einen beliebigen pharmazeutischen Träger, der selbst nicht die Produktion von Antikörpern auslöst, die für das Individuum, dem die Rezeptur verabreicht wurde, gefährlich sein könnten, und der keine unangemessene Giftigkeit besitzen. Geeignete "pharmazeutisch akzeptable Träger" können große, langsam abbaubare Makromoleküle, wie beispielsweise Proteine, Polysaccharide, Polylactonsäuren, Polyglykolsäuren, polymere Aminosäuren, Aminosäure-Kopolymere und inaktivierte Virusbestandteile sein. Solche Träger sind dem Fachmann wohlbekannt.

Salze der Peptide oder funktionell äquivalenter Verbindungen können mit bekannten Methoden hergestellt werden, was typischer Weise bedeutet, dass die Peptide, Peptidomimetika, Peptid-Konjugate oder Peptidomimetika-Konjugate mit einer pharmazeutisch akzeptablen Säure zu einem sauren Salz oder mit einer pharmazeutisch akzeptablen Base zu einem basischen Salz gemischt werden. Ob eine Säure oder eine Base pharmazeutisch akzeptabel sind, kann leicht von einem Fachmann in Kenntnis der Anwendung und der Rezeptur festgelegt werden. So sind beispielsweise nicht alle Säuren und Basen, die für *ex vivo* Anwendungen akzeptabel sind, auch auf therapeutische Rezepturen übertragbar. Abhängig von der jeweiligen Anwendung können pharmazeutisch akzeptable Säuren sowohl organischer als auch anorganischer Natur sein, z.B. Ameisensäure, Essigsäure, Propionsäure, Milchsäure, Glykolsäure, Oxalsäure, Brenztraubensäure, Bernsteinsäure, Maleinsäure, Malonsäure, Zimtsäure, Schwefelsäure, Salzsäure, Bromwasserstoffsäure, Salpetersäure, Perchlorsäure, Phosphorsäure und Thiocyansäure, die mit den freien Aminogruppen von Peptiden und funktionell äquivalenten Verbindungen Ammoniumsalze ausbilden. Pharmazeutisch akzeptable Basen, die mit freien Carbonsäuregruppen der Peptide und funktionell äquivalenten Verbindungen Carboxylate ausbilden, beinhalten Ethylamin, Methylamin, Dimethylamin, Triethylamin, Isopropylamin, Diisopropylamin und andere Mono-, Di- und Trialkylamine sowie Arylamine. Ferner sind pharmazeutisch akzeptable Lösungsmittel eingeschlossen.

Pharmazeutisch akzeptable Salze können darin zur Anwendung kommen, wie z. B. Salze von Mineralsäuren, wie Hydrochloride, Hydrobromide, Phosphate, Sulfate und vergleichbare; aber auch Salze organischer Säuren, wie Acetate, Propionate, Malonate, Benzoate und vergleichbare. Eine ausführliche Diskussion zur pharmazeutisch akzeptablen Inhaltsstoffen findet man in Remington's Pharmaceutical Sciences (Mack Pub. Co., N.J., 1991).

Pharmazeutisch akzeptable Träger in den therapeutischen Zusammensetzungen können Flüssigkeiten enthalten, beispielsweise Wasser, Salzwasser, Glycerol und Ethanol. Zusätzlich können Hilfsstoffe zugegeben werden, wie Befeuchtungsmittel oder Emulgatoren, pH puffernde Substanzen und ähnliche Verbindungen können in solchen Mitteln vorhanden sein. Typischer Weise werden die therapeutischen Zusammensetzungen entweder in flüssiger Form oder als Suspension zur Injektion zubereitet, feste Formen zum Auflösen oder Suspendieren in Trägerflüssigkeiten vor der Injektion sind ebenfalls möglich. Liposomen sind auch in der Definition eines "pharmazeutisch akzeptablen Trägers" enthalten.

Zur therapeutischen Behandlung können modifizierte Peptide oder Peptid-Konjugate, wie oben beschrieben, produziert und einem Subjekt, das diese benötigt, verabreicht werden. Das Peptid oder Peptid-Konjugat kann einem Subjekt in beliebiger, geeigneter Form verabreicht werden, vorzugsweise als pharmazeutische Zusammensetzung, die der Darreichungsform angepasst ist und in einer für die angestrebte Behandlung angemessenen Dosierung vorliegt.

Die pharmazeutischen Zusammensetzungen dieser Erfindung können weitere aktive Verbindungen enthalten, beispielsweise konventionelle Antibiotika (z.B. Vancomycin, Streptomycin, Tetracyclin, Penicillin) oder andere antimikrobiell aktive Verbindungen, wie Fungizide, z.B. Intraconazol oder Myconazol. Auch andere Verbindungen, die mit der Infektion einhergehende Symptome wie Fieber (Salizylsäure) oder Hautausschlag lindern, können zugesetzt werden.

Neben der therapeutischen Verwendung zur Behandlung von Infektionen, oder auch bei der biologischen Kriegsführung, ist es weiter möglich, die erfindungsgemäßen Peptide oder Peptidderivate in Desinfektions- oder Reinigungsmitteln (z. B. einer bakterioziden Zusammensetzung) einzusetzen, die zur Desinfektion oder Reinigung von Oberflächen oder Gegenständen verwendet werden können. Ein anderes Anwendungsgebiet sind Verpackungen, wo Peptide an Verpackungsmaterial gebunden oder darin eingebunden werden können, oder als Konservierungsmittel für andere Materialien, die leicht durch Mikroorganismen abgebaut werden können. Die erfindungsgemäßen Peptide oder Peptidderivate sind insbesondere zur Verpackung von Lebensmitteln geeignet, da sie weder beim Kontakt noch bei der Aufnahme toxisch wirken.

Ein anderer Bestandteil dieser Erfindung ist eine Methode zur Behandlung von Säugetieren, die mit Mikroben (insbesondere Bakterien oder Pilze) infiziert sind, einschließlich der Verabreichung einer effektiven, therapeutisch wirksamen Menge der pharmazeutisch wirksamen erfindungsgemäßen Zusammensetzung.

Der hier verwendete Begriff "therapeutisch wirksame Menge" bezeichnet die Menge eines Therapeutikums, d. h. eines erfindungsgemäßen Peptids, Peptidomimetikums, Peptid-Konjugats oder Peptidmimetika-Konjugats, welche die Vermehrung und Kolonienbildung der Bakterien zu reduzieren oder ganz zu verhindern oder einen messbaren therapeutischen bzw. prophylaktischen Erfolg zu erzielen vermag. Der Effekt kann beispielsweise für Biopsien in Kultur, durch Test der bakteriellen Aktivität oder mit einer anderen geeigneten Methode zur Beurteilung des Umfangs und des Grads einer bakteriellen Infektion bestimmt werden. Die genaue effektive Menge für ein Subjekt hängt von dessen Größe und Gesundheitszustand, der Art und dem Ausmaß der Erkrankung und den Therapeutika oder der Kombination mehrerer Therapeutika ab, die zur Behandlung ausgewählt wurden. Insbesondere die erfindungsgemäßen Zusammensetzungen können verwendet werden, um baktierielle Infektionen und/oder biologische oder physische Begleiterscheinungen (z.B. Fieber) zu reduzieren oder zu verhindern. Methoden zur Festlegung der Anfangsdosis durch einen Mediziner sind Stand der Technik. Die festgelegten Dosen müssen sicher und erfolgreich sein.

Die Menge eines erfindungsgemäßen Proteins, Peptids oder Nukleinsäure, die für eine antibakteriell effektive Dosis notwendig ist, kann unter Berücksichtigung des Pathogens, das die Infektion auslöst, der Schwere der Infektion, sowie vom Alter, Gewicht, Geschlecht, allgemeiner physischer Kondition usw. des Patienten festgelegt werden. Die notwendige Menge der aktiven Komponente, um effektiv antibakteriell und antimykotisch ohne nennenswerte Nebenwirkungen wirksam zu sein, hängt von der eingesetzten pharamazeutischen Rezeptur und der eventuellen Anwesenheit weiterer Bestandteile wie. Antibiotika, Antimykotika usw. ab. Für die erfindungsgemäßen Einsatzgebiete kann eine effektive Dosis zwischen 0,01 nmol/kg und 50 nmol/kg liegen, vorzugsweise zwischen 0,2 nmol/kg und 10 nmol/kg des Peptids, Peptidomimetikas, Pepid-Konjugats oder Peptidomimetik-Konjugats in dem behandelten Individuum.

Anfangsdosen der erfindungsgemäßen Peptide, Peptidomimetika, Multimere, Peptid-Konjugate oder Peptidomimetika-Konjugate können optional durch wiederholte Verabreichung verfolgt werden. Die Häufigkeit der Dosierungen hängt von den oben identifizierten Faktoren ab und beträgt vorzugsweise zwischen ein und sechs Dosen pro Tag über einen Behandlungszeitraum von etwa drei Tagen bis maximal einer Woche.

In einer weiteren Ausführungsform werden die Verbindungen pulmunal in einer bestimmten Menge verabreicht, z.B. durch einen Inhalator, Zerstäuber, Aerosolspray oder einen Inhalator für trockenes Pulver. Geeignete Formulierungen können durch bekannte Methoden und Techniken hergestellt werden. Eine transdermale oder rektale Zufuhr mag in einigen Fällen genau wie die Verabreichung in das Auge angebracht sein.

Es kann vorteilhaft sein, die erfindungsgemäßen Substanzen durch fortgeschrittene Formen der Arzneimittelgabe (advanced drug delivery or targeting methods) effektiver zu verabreichen. So kann, falls der Verdauungstrakt umgangen werden soll, die Darreichungsform eine beliebige Substanz oder Mischung enthalten, die die Bioverfügbarkeit steigert. Dies kann beispielsweise dadurch erreicht werden, dass der Abbau reduziert wird, z.B. durch einen Enzyminhibitor oder ein Antioxidants. Besser ist es, wenn die Bioverfügbarkeit der Verbindung durch eine Zunahme der Permeabilität der Absorptionsbarriere, meist die Schleimhaut, erzielt wird. Substanzen, die das Eindringen erleichtern, können auf verschiedene Arten wirken; einige erhöhen die Fluidität der Schleimhaut, während andere die Zwischenräume zwischen den Schleimhautzellen erweitern. Wiederum andere reduzieren die Viskosität des Schleims auf der Schleimhaut. Zu den bevorzugten Aufnahmebeschleunigern gehören amphiphile Substanzen wie Cholinsäurederivate, Phospholipide, Ethanol, Fettsäuren, Ölsäure, Fettsäurederivate, EDTA, Carbomere, Polycarbophil und Chitosan.

Indikationen, für welche die erfindungsgemäßen modifizierten Peptide, deren Konjugate oder Multimere eingesetzt werden können, sind bakterielle Infektionen mit sowohl Gram-positiven als auch Gram-negativen Bakterien, beispielsweise *Escherichia coli, Enterobacter cloacae, Erwinia amylovora, Klebsiella pneumoniae, Morganella morganii, Pseudomonas aeruginosa, Salmonella typhimurium, Salmonella typhi, Shigella dysenteriae, Yersinia enterocolitica, Acinetobacter calcoaceticus, Agrobacterium tumefaciens, Francisella tularensis, Legionella pneumophila, Pseudomonas syringae, Rhizobium meliloti, Haemophilus influenzae.*
Die Erfindung wird nachfolgend durch folgende Ausführungsbeispiele und Figuren erläutert, ohne die Erfindung auf diese zu beschränken:

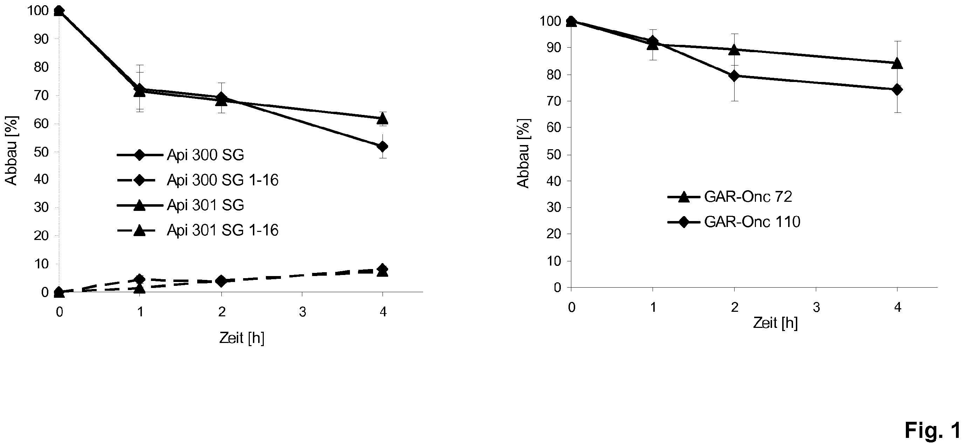

**Fig. 1** zeigt die Serumstabilität von Api 300 (SG), Api 301 (SG) (links), Onc 72 und Onc110 (rechts) in Mausserum (100 %). Gezeigt werden die jeweiligen Ausgangsprodukte (durchgezogene Linie) und die Abbauprodukte (gestrichelte Linie) sofern diese detektiert wurden.

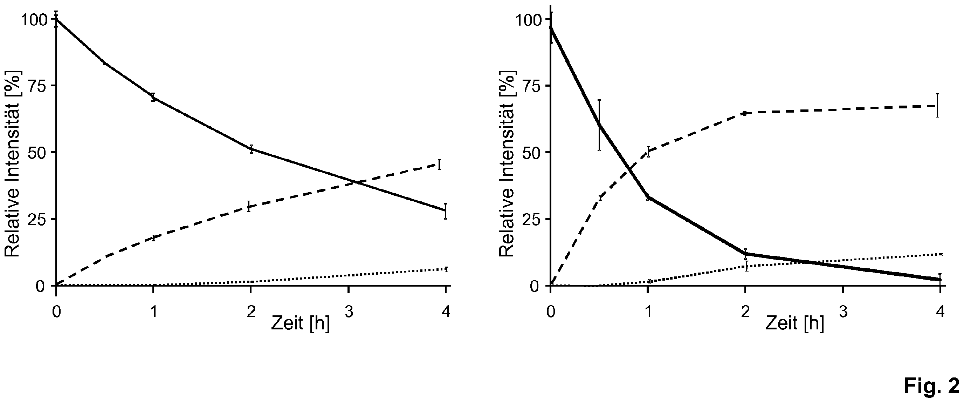

**Fig. 2** zeigt die Serumstabilität von Api 137 (sPEG⁷⁵⁰-GRSG) (links, durchgezogene Linie) und Api 137 (PEG⁷⁵⁰-GARSG) (rechts, durchgezogene Linie) in 25% wässrigem Mausserum (100 %). Der jeweils freigesetzte Wirkstoff Api 137 (SG) (gestrichelte Linie) und das daraus entstehende, am C-Terminus verkürzte Abbauprodukt Api 137 (SG) 1-16 (gepunktete Linie).

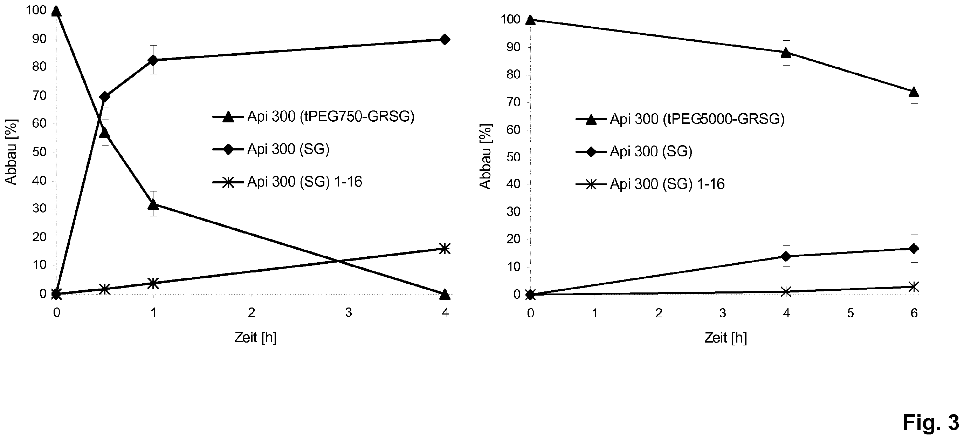

**Fig. 3** zeigt die Serumstabilität von Api 300 (tPEG⁷⁵⁰-GRSG) (links) und Api 300 (tPEG⁵⁰⁰⁰-GRSG) (rechts) in Mausserum (100 %). Gezeigt werden die jeweiligen Ausgangsprodukte (Dreieck), der freigesetzte Wirkstoff Api 300 (SG) (Raute) und dessen am C-Terminus verkürztes Abbauprodukt Api300 (SG) 1-16 (Stern).

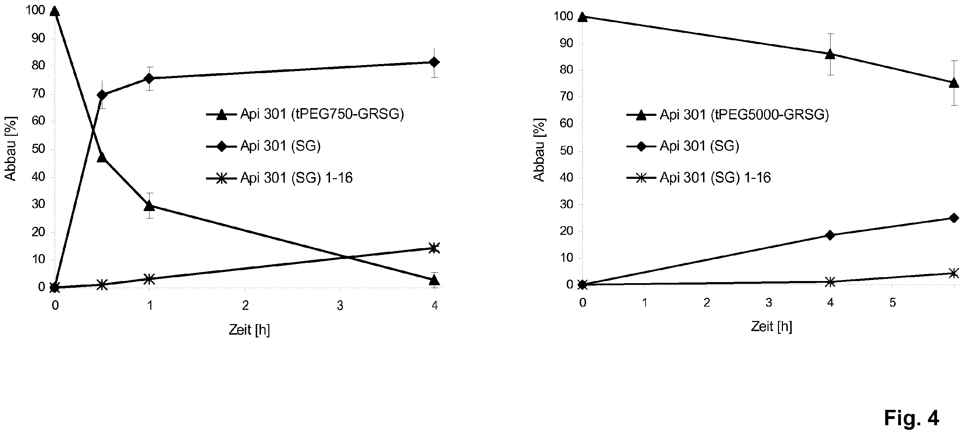

**Fig. 4** zeigt die Serumstabilität von Api 301 (tPEG⁷⁵⁰-GRSG) (links) und Api 301 (tPEG⁵⁰⁰⁰-GRSG) (rechts) in Mausserum (100 %). Gezeigt werden die jeweiligen Ausgangsprodukte (Dreieck), der freigesetzte Wirkstoff Api 301 (SG) (Raute) und dessen am C-Terminus verkürztes Abbauprodukt Api301 (SG) 1-16 (Stern).

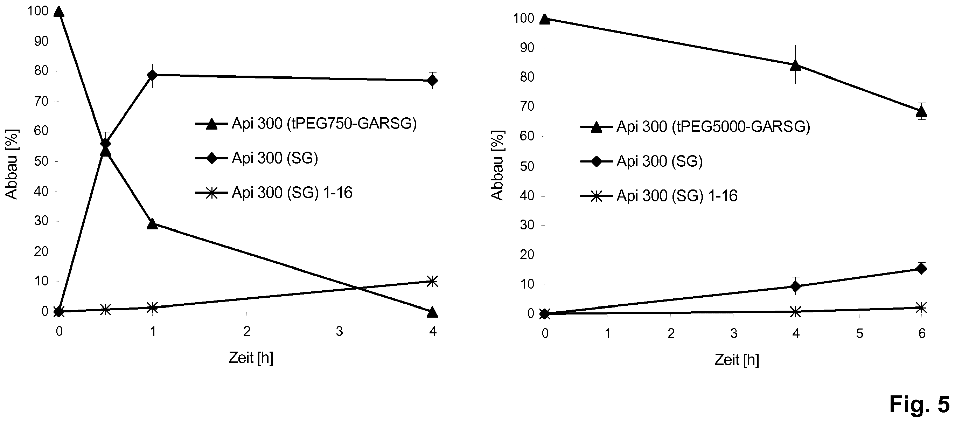

**Fig. 5** zeigt die Serumstabilität von Api 300 (tPEG⁷⁵⁰-GARSG) (links) und Api 300 (tPEG⁵⁰⁰⁰-GARSG) (rechts) Mausserum (100 %). Gezeigt werden die jeweiligen Ausgangsprodukte (Dreieck), der freigesetzte Wirkstoff Api 300 (SG) (Raute) und dessen am C-Terminus verkürztes Abbauprodukt Api300 (SG) 1-16 (Stern).

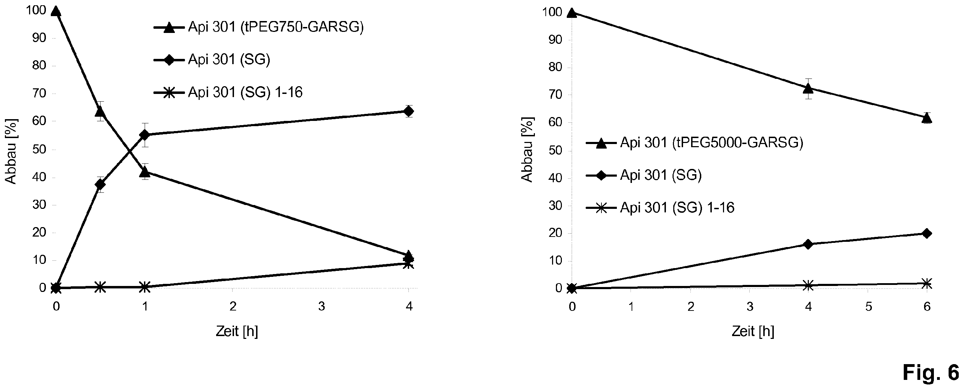

**Fig. 6** zeigt die Serumstabilität von Api 301 (tPEG⁷⁵⁰-GARSG) (links) und Api 301 (tPEG⁵⁰⁰⁰-GARSG) (rechts) in Mausserum (100 %). Gezeigt werden die jeweiligen Ausgangsprodukte (Dreieck), der freigesetzte Wirkstoff Api 301 (SG) (Raute) und dessen am C-Terminus verkürztes Abbauprodukt Api301 (SG) 1-16 (Stern).

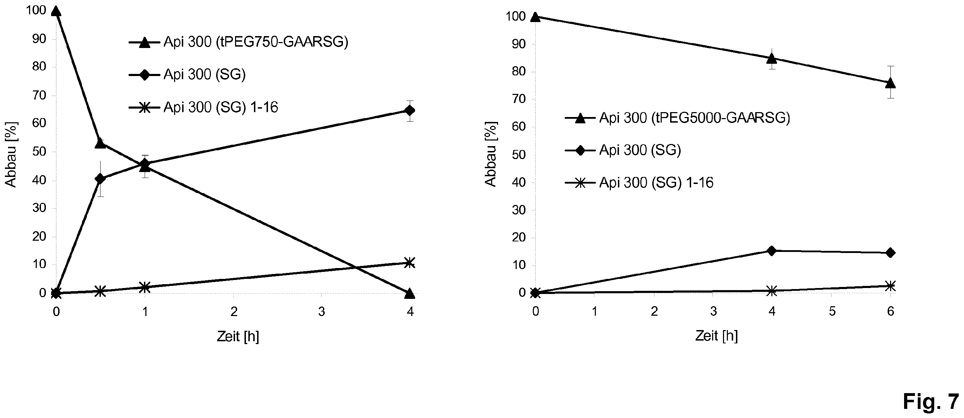

**Fig. 7** zeigt die Serumstabilität von Api 300 (tPEG⁷⁵⁰-GAARSG) (links) und Api 300 (tPEG⁵⁰⁰⁰-GAARSG) (rechts) Mausserum (100 %). Gezeigt werden die jeweiligen Ausgangsprodukte (Dreieck), der freigesetzte Wirkstoff Api 300 (SG) (Raute) und dessen am C-Terminus verkürztes Abbauprodukt Api300 (SG) 1-16 (Stern).

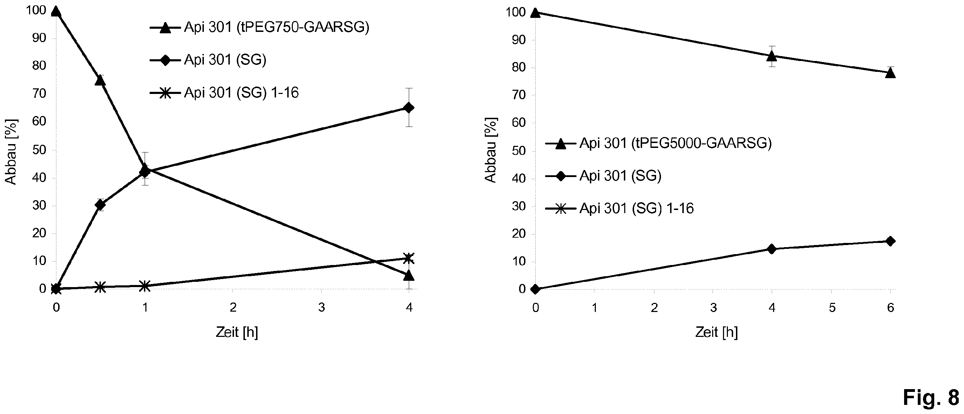

**Fig. 8** zeigt die Serumstabilität von Api 301 (tPEG⁷⁵⁰-GAARSG) (links) und Api 301 (tPEG⁵⁰⁰⁰-GAARSG) (rechts) in Mausserum (100 %). Gezeigt werden die jeweiligen Ausgangsprodukte (Dreieck), der freigesetzte Wirkstoff Api 301 (SG) (Raute) und dessen am C-Terminus verkürztes Abbauprodukt Api301 (SG) 1-16 (Stern).

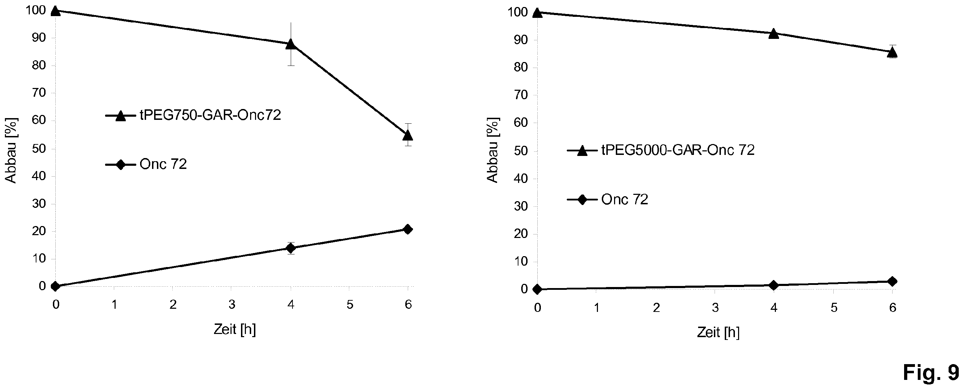

**Fig. 9** zeigt die Serumstabilität von tPEG⁷⁵⁰-GAR-Onc 72 (links) und tPEG⁵⁰⁰⁰-GAR-Onc 72 (rechts) in Mausserum (100 %). Gezeigt werden die jeweiligen Ausgangsprodukte (Dreieck) und der freigesetzte Wirkstoff Onc72 (Raute). Abbauprodukte von Onc72 wurden nicht detektiert.

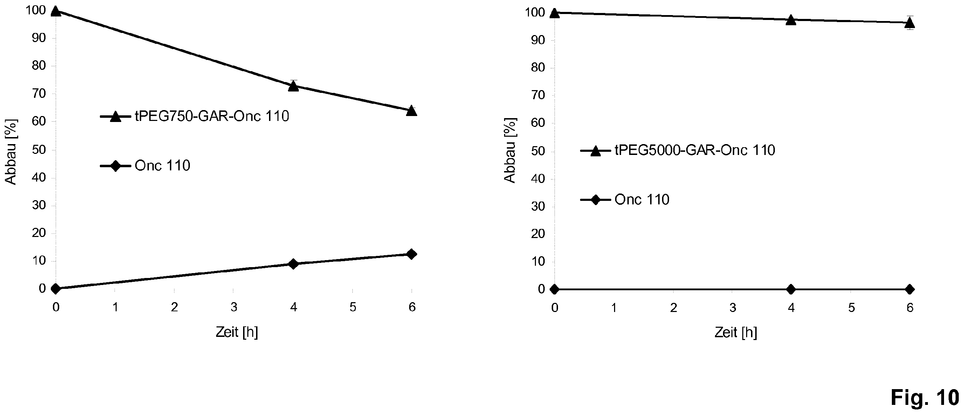

**Fig. 10** zeigt die Serumstabilität von tPEG⁷⁵⁰-GAR-Onc 110 (links) und tPEG⁵⁰⁰⁰-GAR-Onc 110 (rechts) in Mausserum (100 %). Gezeigt werden die jeweiligen Ausgangsprodukte (Dreieck) und der freigesetzte Wirkstoff Onc 110 (Raute). Abbauprodukte von Onc 110 wurden nicht detektiert.

### Beispiel 1 Peptidsynthese und Modifizierung

### 1. Peptidsynthese:

Alle Chemikalien für die Peptidsynthese wurden, wenn nicht anders angegeben, von Fluka Chemie GmbH (Buchs, Schweiz) in der größtmöglichen Reinheit bezogen.

Die Peptide wurden mittels konventioneller Festphasenpeptidsynthese unter Verwendung der Fmoc/^{t}Bu-Strategie synthetisiert. Alle Standard-Fmoc-Aminosäuren wurden von MultiSynTech GmbH (Witten, Deutschland) bzw. Orpegen Pharma GmbH (Heidelberg, Deutschland) bezogen. Trans-4-Hydroxyprolin (t-4-Hyp) und tert.-Leucin wurde von Novabiochem (Merck Biosciences GmbH, Darmstadt, Deutschland) bezogen.

Die Peptide wurden im 25-µmol-Maßstab mit Hilfe eines SYRO 2000 Peptidsynthese-Roboters (MultiSynTech GmbH, Witten, Deutschland) synthetisiert. Dafür wurden pro Synthesereaktor 42 mg Leu-Wang-Harz (Beladung: 0,6 mmol/g) eingewogen und 30 min in DMF gequollen. Nach der Abspaltung der N-terminalen Fmoc-Schutzgruppe wurden die Peptide mit dem nachfolgenden Synthesezyklus (Tab. 2) synthetisiert.

**Tabelle 2: Synthesezyklus der automatischen multiplen Festphasenpeptidsynthese (SYRO 2000) für die Kopplung einer Aminosäure.**

| **Syntheseschritt** | **Reagenzien** | **Reaktionszeit** |
|---|---|---|
| Aminosäurekopplung | 8 eq. Aminosäure in HOBt/DMF (0,5 mol/L) und DIC/DMF (2,0 mol/L) | 60 min |
| Waschen | 3 x mit je 600 µL DMF | 1 min |
| Fmoc-Abspaltung | 400 µL 40 % Piperidin/DMF | 3 min |
| | 500 µL 20 % Piperidin/DMF | 10 min |
| Waschen | 6 x mit je 600 µL DMF | 1 min |

Für die Synthese im 100-µmol-Maßstab mit Hilfe eines Liberty Mikrowellen-Peptidsynthese-Roboters (CEM GmbH, Kamp-Lintfort, Deutschland) wurden pro Synthesereaktor 156,3 mg kommerziell erhältliches Leu-Wang-Harz der Firma NovaBiochem (Beladung: 0,64 mmol/g) eingewogen und 30 min in DMF gequollen. Nach der Abspaltung der N-terminalen Fmoc-Schutzgruppe, wurden die Peptide u. a. mit dem nachfolgenden Synthesezyklus (Tab. 3) synthetisiert.

**Tabelle 3: Synthesezyklus der automatischen multiplen Festphasenpeptidsynthese (Liberty) für die Kopplung der einzelnen Aminosäuren.**

| **Zyklus** | **Syntheseschritt** | **Reagenzien** | **Reaktionszeit** |
|---|---|---|---|
| Aminosäure | Waschen | 7.0 mL DMF | |
| (0,10-Einzel) | Fmoc-Abspalten | 7.0 mL 20 % Piperidin | 0.5 min |
| | | 35 Watt bei 75 °C | |
| | Waschen | 5.0 mL DMF | |
| | Fmoc-Abspalten | 7.0 mL 20 % Piperidin | 3 min |
| | | 35 Watt bei 75 °C | |
| | Waschen | 4 x mit je 7.0 mL DMF | |
| | Kopplung | 2.5 mL 0.2 mol/L Aminosäure in DMF | 10 min |
| | | 1.0 mL 0.5 mol/L HBTU | |
| | | 25 Watt bei 75 °C | |
| | Waschen | 3 x mit je 7.0 mL DMF | |

Die Peptide wurden entweder als Säure an Wang-Harz bzw. Leu-Wang-Harz oder als Säureamid an Rink-Amid 4-Methylbenzylhydrylamin (MBHA) Harz (0,67 mmol/g), der Firma MultiSynTech GmbH (Witten, Deutschland) synthetisiert.
Als Seitenkettenschutzgruppen wurden Triphenylmethyl (trityl) für Cys, Asn, His und Gln, *tert.-*Butylether (^{t}Bu) für Tyr, Ser und Thr, *tert*.-Butylester (O^{t}Bu) für Asp und Glu, ω-N-2,2,4,6,7-Pentamethyldihydrobenzofuran-5-sulfonyl (Pbf) für Arg, tert.-Butyloxy-carbonyl (Boc) für Lys und Orn eingesetzt. Die temporäre Fmoc-Schutzgruppe wurde mit 40% Piperidin (Biosolve BV, Valkenswaard, Niederlande) in DMF (v/v) für 5 min und erneut mit frischen 20% Piperidin in DMF (v/v) für 10 min abgespalten.

### 2. Modifizierung der Peptide:

### a.) Guanidierung des N-Terminus:

Das Harz (1 eq.) wurde zunächst für 20 min in DMF gequollen. Dann wurde es mit HBTU in DMF (0,5 mol/L, 10 eq.) und NMM (10 eq.) für 3 Stunden bei Raumtemperatur und unter Schütteln inkubiert. Dann wurde die Reaktionslösung erneuert und die Suspension für weitere 4 Stunden inkubiert. Nach Abschluss der Reaktion wurde die Reaktionslösung abgetrennt und das Harz sechsmal mit DMF bzw. DCM gewaschen.

### b.) Iodacetylierung

Das Harz (1 eq) wurde zunächst für 20 min in DMF gequollen. Dann wurde es mit Iodessigsäure (8 eq.) und HOBt in DMF (0,5 mol/L, 8 eq.) versetzt und die Reaktion mit DIC (8 eq.) aktiviert. Anschließend wurde das Reaktionsgemisch für 16 Stunden bei Raumtemperatur im Dunkeln und unter Schütteln inkubiert. Nach Abschluss der Reaktion wurde die Reaktionslösung abgetrennt und das Harz sechsmal mit DMF bzw. DCM gewaschen und anschließend getrocknet.

Die Vollständigkeit der Modifikation am N-terminus des Peptids bzw. der Seitenkette wurde mittels Kaisertest überprüft. Dazu wurde etwas Harz mit 0,28 mol/L Ninhydrin (Riedel de Haen, Seelze, Deutschland) in Ethanol (Carl Roth GmbH + Co. KG, Karlsruhe, Deutschland), 0,2 mmol/L Kaliumcyanid in Pyridin und 76% Phenol in Ethanol im Verhältnis (1:1:2) bei 95°C inkubiert. Falls eine Blaufärbung auftrat, was auf freie primäre Aminogruppen hindeutet, wurde die Kopplung wiederholt.

### 3. Kopplung des Polyethylenglykols:

Da das Polyethylenglykol hygroskopische Eigenschaften aufweist, wurde es vor jeder Kopplungsreaktion am Hochvakuum getrocknet.

Folgende Polyethylenglykole-Derivate (alle Iris Biotech GmbH, Marktredwitz, Deutschland, > 95 % Reinheit) wurden als Ausgangsstoffe für die nachfolgend beschreibenen Kopplungen verwendet:

| | |
|---|---|
| α-Methoxy-ω-carbonsäure Polyethylenglykol (20000 Da) | MeO-PEG²⁰⁰⁰⁰-COOH |
| α-Methoxy-ω-carbonsäure Polyethylenglykol) (750 Da) | MeO-PEG⁷⁵⁰-COOH |
| α-Methoxy-ω-carbonsäure Polyethylenglykol) (5000 Da) | MeO-PEG⁵⁰⁰⁰-COOH |
| α-Methoxy-ω-mercapto Polyethylenglykol (750 Da) | MeO-PEG⁷⁵⁰-SH |
| α-Methoxy-ω-mercapto Polyethylenglykol (5000 Da) | MeO-PEG⁵⁰⁰⁰-SH |

### a.) Kopplung des Polyethylenglykols mit freier Säuregruppe (sPEG, z. B. für MeO-PEG-COOH) via DIC und HOBt:

Das Harz (1 eq.) wurde zunächst für 20 min in DMF gequollen. Dann wurde es mit dem in HOBt/DMF (0,5 mol/L, 5 eq.) gelösten Polyethylenglykol-Derivat (5 eq.) versetzt und die Reaktion mit DIC (5 eq.) aktiviert. Anschließend wurde das Reaktionsgemisch für eine Stunde bei RT und unter Schütteln inkubiert. Dann wurde erneut DIC (2,5 eq.) beigefügt und für weitere 30 min inkubiert. Nach Abschluss der Reaktion wurde die Reaktionslösung abgetrennt und das Harz sechsmal mit DMF gewaschen. Die vollständige Kopplung wurde mittels Kaiser-Test überprüft.

### b.) Kopplung nach Aktivierung des Polyethylenglykol, insbesondere für PEG⁵⁰⁰⁰ und größer:

Hier wurde das Polyethylenglykol-Derivat mit freier Säuregruppe zunächst als N-Hydroxysuccinimid-(NHS-)Ester aktiviert und anschließend in Lösung gekoppelt.

Zur Bildung des Aktivesters wurden das Polyethylenglykol (1 eq.) mit je einem 1 eq. des zuvor in DMF gelösten DCC und N-Hydroxysuccinimid bei Raumtemperatur unter Schütteln inkubiert bis sich nach drei Stunden ein feiner Niederschlag bildete. Der im Überstand gelöste PEG-Aktivester (1 eq.) wurde zum Peptid (0,9 eq.) gegeben und bei RT unter Schütteln in PBS (pH = 7,4) bzw. DMF inkubiert. Die Reaktion wurde mittels RP-HPLC und MALDI-TOF-MS bzw. mit LC-MS verfolgt.

### c.) Thioether-Ligation mit Thiol-modifziertem Polyethylenglykol (tPEG):

Das iodacetylierte Peptid (4 eq.) in PBS (pH = 7,4) wurde mit einem Thiolderivatsierten Polyethylenglykol (z. B. MeO-PEG-SH) (1 eq.) versetzt, die Reaktionslösung entgast und unter einer N₂-Atmosphäre bei 4 °C für 18 Stunden inkubiert. Der Fortschritt der Reaktion wurde mittels RP-HPLC und MALDI-TOF-MS verfolgt.

Die PEGylierten Peptide wurden in einer Ausbeute von 25 bis 40 % erhalten und mittels RP-HPLC und MALDI-MS charakterisiert.

**Tabelle 4: Übersicht der synthetisierten Peptidsequenzen**

| SEQ ID No. | Name | Sequenz |
|---|---|---|
| 2 | Api 1b* | GNNRPVYIPQPRPPHPRL-OH |
| 14 | Api 88* | Guan-ONNRPVYIPRPRPPHPRL-NH₂ |
| 15 | Api 134* | Guan-ONNRPVYIPRPRPPHPOL-NH₂ |
| 16 | Api 137* | Guan-ONNRPVYIPRPRPPHPRL-OH |
| 17 | Api 137 (sPEG⁷⁵⁰) | Guan-O(sPEG⁷⁵⁰) NNRPVYIPRPRPPHPRL-OH |
| 18 | Api 137 (SG)* | Guan-O(SG)NNRPVYIPRPRPPHPRL-OH |
| 19 | Api 137 (GRSG)* | Guan-O(GRSG)NNRPVYIPRPRPPHPRL-OH |
| 20 | Api 137 (GARSG)* | Guan-O(GARSG)NNRPVYIPRPRPPHPRL-OH |
| 21 | Api 137 (sPEG⁷⁵⁰-GRSG) | Guan-O(sPEG⁷⁵⁰-GRSG) NNRPVYIPRPRPPHPRL-OH |
| 22 | Api 137 (sPEG⁷⁵⁰-GARSG) | Guan-O(sPEG⁷⁵⁰-GARSG) NNRPVYIPRPRPPHPRL-OH |
| 23 | Api 137 (sPEG⁵⁰⁰⁰-GARSG) | Guan-O(sPEG⁵⁰⁰⁰-GARSG) NNRPVYIPRPRPPHPRL-OH |
| 24 | Api 137 (sPEG²⁰⁰⁰⁰-GARSG) | Guan-O (sPEG²⁰⁰⁰⁰-GARSG) NNRPVYIPRPRPPHPRL-OH |
| 25 | Api 300 (SG)⁺ | Guan-OO(SG)NRPVYIPRPRPPHPRL-OH |
| 26 | Api 300 (GRSG)* | Guan-OO(GRSG)NRPVYIPRPRPPHPRL-OH |
| 27 | Api 300 (GARSG)* | Guan-OO(GARSG)NRPVYIPRPRPPHPRL-OH |
| 28 | Api 300 (GAARSG)* | Guan-OO(GAARSG)NRPVYIPRPRPPHPRL-OH |
| 29 | Api 300 (sPEG⁷⁵⁰-GRSG) | Guan-OO(sPEG⁷⁵⁰-GRSG)NRPVYIPRPRPPHPRL-OH |
| 30 | Api 300 (sPEG⁷⁵⁰-GARSG) | Guan-OO(sPEG⁷⁵⁰-GARSG)NRPVYIPRPRPPHPRL-OH |
| 31 | Api 300 (sPEG⁷⁵⁰-GAARSG) | Guan-OO(sPEG⁷⁵⁰-GAARSG)NRPVYIPRPRPPHPRL-OH |
| 32 | Api 300 (tPEG⁷⁵⁰-GRSG) | Guan-OO(tPEG⁷⁵⁰-GRSG) NRPVYIPRPRPPHPRL-OH |
| 33 | Api 300 (tPEG⁷⁵⁰-GARSG) | Guan-OO(tPEG⁷⁵⁰-GARSG)NRPVYIPRPRPPHPRL-OH |
| 34 | Api 300 (tPEG⁷⁵⁰-GAARSG) | Guan-OO(tPEG⁷⁵⁰-GAARSG)NRPVYIPRPRPPHPRL-OH |
| 35 | Api 300 (tPEG⁵⁰⁰⁰-GRSG) | Guan-OO(tPEG⁵⁰⁰⁰-GRSG) NRPVYIPRPRPPHPRL-OH |
| 36 | Api 300 (tPEG⁵⁰⁰⁰-GARSG) | Guan-OO(tPEG⁵⁰⁰⁰-GARSG) NRPVYIPRPRPPHPRL-OH |
| 37 | Api 300 (tPEG⁵⁰⁰⁰-GAARSG) | Guan-OO(tPEG⁵⁰⁰⁰-GAARSG) NRPVYIPRPRPPHPRL-OH |
| 38 | Api 301 (SG)⁺ | Guan-ONO(SG)RPVYIPRPRPPHPRL-OH |
| 39 | Api 301 (GRSG)* | Guan-ONO(GRSG)RPVYIPRPRPPHPRL-OH |
| 40 | Api 301 (GARSG)* | Guan-ONO(GARSG)RPVYIPRPRPPHPRL-OH |
| 41 | Api 301 (GAARSG)* | Guan-ONO(GAARSG)RPVYIPRPRPPHPRL-OH |
| 42 | Api 301 (sPEG⁷⁵⁰-GRSG) | Guan-ONO(sPEG⁷⁵⁰-GRSG) RPVYIPRPRPPHPRL-OH |
| 43 | Api 301 (sPEG⁷⁵⁰-GARSG) | Guan-ONO(sPEG⁷⁵⁰-GARSG) RPVYIPRPRPPHPRL-OH |
| 44 | Api 301 (sPEG⁷⁵⁰-GAARSG) | Guan-ONO(sPEG⁷⁵⁰-GAARSG)RPVYIPRPRPPHPRL-OH |
| 45 | Api 301 (tPEG⁷⁵⁰-GRSG) | Guan-ONO(tPEG⁷⁵⁰-GRSG) RPVYIPRPRPPHPRL-OH |
| 46 | Api 301 (tPEG⁷⁵⁰-GARSG) | Guan-ONO(tPEG⁷⁵⁰-GARSG) RPVYIPRPRPPHPRL-OH |
| 47 | Api 301 (tPEG⁷⁵⁰-GAARSG) | Guan-ONO(tPEG⁷⁵⁰-GAARSG) RPVYIPRPRPPHPRL-OH |
| 48 | Api 301 (tPEG⁵⁰⁰⁰-GRSG) | Guan-ONO(tPEG⁵⁰⁰⁰-GRSG) RPVYIPRPRPPHPRL-OH |
| 49 | Api 301 (tPEG⁵⁰⁰⁰-GARSG) | Guan-ONO(tPEG⁵⁰⁰⁰-GARSG) RPVYIPRPRPPHPRL-OH |
| 50 | Api 301 (tPEG⁵⁰⁰⁰-GAARSG) | Guan-ONO(tPEG⁵⁰⁰⁰-GAARSG) RPVYIPRPRPPHPRL-OH |
| 51 | Onc 72⁺ | VDKPPYLPRPRPPROIYNO-NH₂ |
| 52 | Onc 110⁺ | VDKPPYLPRPRPHypRHypTleYNO-NH₂ |
| 53 | GAR-Onc 72* | GAR-VDKPPYLPRPRPPROIYNO-NH₂ |
| 54 | GAR-Onc 110* | GAR-VDKPPYLPRPRPHypRHypTleYNO-NH₂ |
| 55 | sPEG⁷⁵⁰-GAR-Onc 72 | sPEG⁷⁵⁰-GAR-VDKPPYLPRPRPPROIYNO-NH₂ |
| 56 | sPEG⁷⁵⁰-GAR-Onc 110 | sPEG⁷⁵⁰-GAR-VDKPPYLPRPRPHypRHypTleYNO-NH₂ |
| 57 | tPEG⁷⁵⁰-GAR-Onc 72* | tPEG⁷⁵⁰-GAR-VDKPPYLPRPRPPROIYNO-NH₂ |
| 58 | tPEG⁷⁵⁰-GAR-Onc 110 | tPEG⁷⁵⁰-GAR-VDKPPYLPRPRPHypRHypTleYNO-NH₂ |
| 59 | tPEG⁵⁰⁰⁰-GAR-Onc 72 | tPEG⁵⁰⁰⁰-GAR-VDKPPYLPRPRPPROIYNO-NH₂ |
| 60 | tPEG⁵⁰⁰⁰-GAR-Onc 110 | tPEG⁵⁰⁰⁰-GAR-VDKPPYLPRPRPHypRHypTleYNO-NH₂ |

| | | |
|---|---|---|
| Guan = Guanidino-Gruppe am N-Terminus, Hyp = *trans*-4-Hydroxyprolin, L(N₃) = Nα-(9-Fluorenylmethyloxycarbonyl)-ε-azido-L-Lysine, O = Ornithin, Pra = Nα-(9-Fluorenylmethyloxycarbonyl)-L-progargylglycine, Tle = L-tertiär-Leucine (L-tertiär -Butylglycine), sPEG: über Säuregruppe verknüpftes PEG, tPEG: über Thiolgruppe verknüpftes PEG. mit * sind Vergleichsbeispiele markiert, mit + sind Spaltungsprodukte markiert. | | |

### c.) Abspaltung Schutzgruppen und Aufreinigung:

Nach Beendigung der Synthese der Peptide und ggf. Modifzierung wurden die Harze sorgfältig mit DMF und DCM gewaschen und getrocknet. Die Harz-gebundenen Peptide wurden mit einer Mischung aus Wasser, m-Kresol, Thioanisol und Ethandithiol (5:5:5:2,5) in 87,5% Trifluoressigsäure (TFA) bei Raumtemperatur für 4 h abgespalten und zugleich die Seitenketten entschützt. Die Peptide und Peptidderivate wurden mit kaltem Diethylether präzipitiert und bei 3000 x g abzentrifugiert. Das Pellet wurde zweimal mit kaltem Ether gewaschen, getrocknet und in 0,1% wässriger TFA (UV-Spektroskopie) gelöst. Die Proben wurden bei -20°C gelagert.

Die abgespaltenen (ggf. modifizierten) Peptide wurden mittels RP-HPLC an einem Äkta HPLC System (Amersham Bioscience GmbH, Freiburg, Deutschland) mit einer Jupiter C₁₈ 5 µm 300 Å, 250 x 10 mm bzw. Jupiter C₁₈ 15 µm, 300 Å, 250 x 21 mm Säule (Phenomenex Inc., Torrance, USA) gereinigt.

Als Laufmittel wurde jeweils 0,1% wässrige TFA (Eluent A) und 60% wässriges Acetonitril (Biosolve BV, Valkenswaard, Niederlande) mit 0,1% TFA (Eluent B) verwendet. Ein typischer linearer Gradient begann bei 5% B und die Elution erfolgte bei einer Steigung von 1% B pro Minute mit einer Flussrate von 10 mL/min (250 x 21 mm Säule) bzw. 5 mL/min (250 x 10 mm Säule). Detektiert wurde bei 220, 230 und 240 nm. Die Analytik der gereinigten Peptide erfolgte mit dem gleichen HPLC System mit einer Jupiter C₁₈ 5 µm, 300 Å, 150 x 4,6 mm Säule (Phenomenex Inc., Torrance, USA). Eluiert wurde bei einer Flussrate von 1 mL/min mit einem linearen Gradienten von 5-95% B in 30 min und detektiert bei 220 nm. Zusätzlich wurde die Reinheit mittels Matrix-unterstützter Laser Desorption/Ionisierung mit Flugzeit-Massenspektrometrie (MALDI-TOF-MS; 4700 Proteomic Analyzer, Applied Biosystems GmbH, Darmstadt, Deutschland) bestimmt. Dafür wurde 0,5 µL Peptidlösung mit 0,5 µL α-Cyanohydroxyzimtsäure (Bruker Daltonik GmbH; Bremen, Deutschland) als Matrix (4 mg/mL in 60% Acetonitril in 0,1% wässriger TFA) co-kristallisiert.

### Beispiel 2: Bestimmung der minimalen Hemmkonzentrationen und Wachstumskinetik

### 1. minimale Hemmkonzentrationen:

Die minimale Hemmkonzentrationen (MHK) der Peptide wurden in einer Doppelbestimmung von Triplikaten mit einer Positiv-(Gentamycin) und einer Negativkontrolle (0,9 % NaCl-Lösung) ermittelt.

Die Peptide wurden dazu in Wasser gelöst und in einer zweifachen Verdünnungsreihe mit 1 % wässrigem Sojabohnen-Medium (TSB) in sterilen 96-Well-Platten (Greiner Bio-One GmbH) von 128 µg/mL in zwölf Verdünnungsschritten auf 62,5 ng/mL verdünnt. Übernachtkulturen von *Escherichia coli* Stamm BL21AI wurden mit 1 % TSB auf ca. 1,5 x 10⁷ Kolonie bildende Einheiten pro mL eingestellt. Daraufhin wurde jeweils 50 µL Peptidlösung pro Well mit je 50 µL der Bakterienlösung vermischt, um eine Anfangskonzentration von 4 x 10⁵ Bakterien pro Well zu erreichen. Nach 20 Stunden Inkubation bei 37 °C wurde die Absorption bei 595 nm (Mikroplatten-Leser, TECAN Trading AG) bestimmt. Die minimale Hemmkonzentration wurde als die niedrigste Peptidkonzentration identifiziert, bei der kein bakterielles Wachstum nachgewiesen wurde.

Zusätzlich wurde die antibakterielle Aktivität auch in Gegenwart von 25% Mausserum bestimmt. Dazu wurden, wie oben beschrieben, die MHK-Werte bestimmt, allerdings wurden jedem Well vor der Inkubation noch 25 µL Mausserum zugesetzt (25% Endkonzentration).

**Tabelle 5: Minimale Hemmkonzentrationen.**

| Seq. ID | Peptidderivat | MHK [µmol/L] in TSB | MHK [µmol/L] in TSB/Mausserum |
|---|---|---|---|
| 2 | Apidaecin 1b* | 0,48 | 1,75 |
| 14 | Api 88* | 0,44 | 1,19 |
| 15 | Api 134* | 1,78 | 7,12 |
| 16 | Api | 0,46 | 0,23 |
| 18 | Api 137 (SG)⁺ | 0,2 | |
| 19 | Api137(GRSG)* | 0,7 | |
| 20 | Api 137 (GARSG)* | 0,9 | |
| 22 | Api 137 (sPEG⁷⁵⁰-GARSG) | 1,1 | |
| 23 | Api 137 (sPEG⁵⁰⁰⁰-GARSG) | 3,7 | |
| 24 | Api 137 (sPEG²⁰⁰⁰⁰-GARSG) | 10,2 | |
| 25 | Api 300 (SG)⁺ | 0,8 | 0,21 |
| 29 | Api 300 (sPEG⁷⁵⁰-GRSG) | 2,9 | 0,37 |
| 30 | Api 300 (sPEG⁷⁵⁰-GARSG) | 2,9 | 0,54 |
| 31 | Api 300 (sPEG⁷⁵⁰-GAARSG) | 2,8 | |
| 32 | Api 300 (tPEG⁷⁵⁰-GRSG) | 2,9 | |
| 33 | Api 300 (tPEG⁷⁵⁰-GARSG) | 1,5 | |
| 34 | Api 300 (tPEG⁷⁵⁰-GAARSG) | 2,8 | |
| 35 | Api 300 (tPEG⁵⁰⁰⁰-GRSG) | >44,8 | |
| 36 | Api 300 (tPEG⁵⁰⁰⁰-GARSG) | 17,4 | 0,44 |
| 37 | Api 300 (tPEG⁵⁰⁰⁰-GAARSG) | 25,4 | |
| 38 | Api 301 (SG)⁺ | 1,6 | |
| 42 | Api 301 (sPEG⁷⁵⁰-GRSG) | 1,5 | |
| 43 | Api 301 (sPEG⁷⁵⁰-GARSG) | 2,9 | 0,27 |
| 44 | Api 301 (sPEG⁷⁵⁰-GAARSG) | 5,6 | |
| 45 | Api 301 (tPEG⁷⁵⁰-GRSG) | 2,9 | |
| 46 | Api 301 (tPEG⁷⁵⁰-GARSG) | 1,5 | |
| 47 | Api 301 (tPEG⁷⁵⁰-GAARSG) | 2,8 | |
| 48 | Api 301 (tPEG⁵⁰⁰⁰-GRSG) | >38 | |
| 49 | Api 301 (tPEG⁵⁰⁰⁰-GARSG) | 9,2 | |
| 50 | Api 301 (tPEG⁵⁰⁰⁰-GAARSG) | >47,5 | |
| 51 | Onc 72⁺ | 1,7 | 1,7 |
| 53 | GAR-Onc 72* | 3,1 | |
| 55 | sPEG750-GAR-Onc 72 | 12,2 | 6,1 |
| 57 | tPEG750-GAR-Onc 72 | 24,3 | |
| 52 | Onc 110⁺ | 6,9 | |
| 54 | GAR-Onc 110* | 1,5 | 0,65 |
| 56 | sPEG750-GAR-Onc 110 | 24,3 | 9,2 |
| 58 | tPEG750-GAR-Onc 110 | 48,4 | |

| | | | |
|---|---|---|---|
| mit * sind Vergleichsbeispiele markiert, mit + sind Spaltungsprodukte markiert. | | | |

Auf Grund der großen Massenunterschiede der unmodifizierten und der PEG-gekoppelten Peptide wird die MHK nicht in der herkömmlichen Einheit µg/mL, sondern in µmol/L angegeben.

Die Ergebnisse zeigen, dass die Anwesenheit des Peptidlinkers (GAR-Onc 72 und GAR-Onc 120) bzw. dessen in den Spaltungsprodukt verbleibenden Reste (Api 137 (SG), Api 300 (SG) und Api 301 (SG)) die MHK nicht oder nur unwesentlich erhöht. In den PEG-modifizierten Peptiden nimmt die MHK in TSB mit der Größe des PEG zu. Ohne Serum (TSB) zeigen die PEG-modifizierten Peptide eine erhöhte MHK. Im Serum haben die PEG-modifizierten Peptide jedoch eine MHK, die mit den unpegylierten Peptiden vergleichbar ist.

### Beispiel 3: Freisetzung der Peptidwirkstoffe in Serum

### 1. Analyse in Maus-Serum:

Es wurden 30 µL einer 1 mg/mL Peptidlösung mit 120 µL Wasser verdünnt und nach Zugabe von 50 µL Serum bei 37 °C für 0 Stunden, 0,5 Stunden, 1 Stunde, 2 Stunden bzw. 4 Stunden inkubiert. Alternativ wurden die Peptide-Derivate mit einer Ausgangskonzentration von 3 mg/mL mit Serum auf eine Endkonzentration von 15 µg/mL verdünnt. Je nach Stabilität der Peptide wurden die Proben zu den zuvor genannten Zeitpunkte analysiert oder nach 0 Stunden, 4 Stunden und 6 Stunden. Die Serumproteine wurden durch Zugabe von 50 µL 15 %iger TCA ausgefällt und der Überstand mit 25 µL einer 1 mol/L NaOH neutralisiert. Es wurden die Proben mit 5 % B auf 250 µL aufgefüllt, 230 µL der Lösung in ein HPLC-Vial überführt und 220 µL injiziert.

Die RP-HPLC Analyse erfolgte mit einem linearen Acetonitrilgradienten (Biosolve BV, Valkenswaard, Niederlande) in Gegenwart von 0,1% Trifluoressigsäure (TFA, UV-grade, Fluka Chemie GmbH, Buchs, Schweitz) als Ionenpaarreagenz. Die Fraktionen wurden mit α-Cyanohydroxyzimtsäure (Bruker Daltonik GmbH; Bremen, Deutschland) als Matrix (4 mg/mL in 60% Acetonitril in 0,1% wässriger TFA) co-kristallisiert und mit einem Tandem-Massenspektrometer (MALDI-TOF/TOF-MS, 4700 Proteomics Analyzer; Applied Biosystems GmbH, Weiterstadt, Deutschland) im Positiv-Ionenreflektor-Modus analysiert. Der Anteil der intakten Peptide und deren Abbauprodukte bzw. Metabolite konnten so zu den einzelnen Zeitpunkten identifiziert und quantifiziert werden. Als Kontrolle diente 25%iges wässriges Mausserum, das parallel für die gleichen Zeitintervalle analysiert wurde.

**Tabelle 6: Übersicht der Halbwertszeiten in 25 %igem (V/V) und reinem Maus-Serum, verglichen wurden die unterschiedlichen Seitenkettenmodifikationen in Kombination mit PEG750; primäre (rot) und sekundäre Spaltstelle (blau), die mit * gekennzeichneten Halbwertszeiten wurden mittels MALDI-TOF MS quantifiziert.**

| Seq. ID | Peptidderivat | Halbwertszeit (t_{½}) | |
|---|---|---|---|
| | | in 25% Serum | in Serum |
| 16 | Api | >360 min | |
| 18 | Api 137 (GRSG)* | 60 min | |
| 20 | Api 137 (GARSG)* | 30 min | |
| 21 | Api 137 (sPEG⁷⁵⁰-GRSG) | 120 min | |
| 22 | Api 137 (sPEG⁷⁵⁰-GARSG) | 60 min | |
| 25 | Api 300 (SG)⁺ | | 240 min |
| 29 | Api 300 (sPEG⁷⁵⁰-GRSG) | | 50 min |
| 32 | Api 300 (tPEG⁷⁵⁰-GRSG) | | 40 min |
| 35 | Api 300 (tPEG⁵⁰⁰⁰-GRSG) | | 75% (6 h)** |
| 30 | Api 300 (sPEG⁷⁵⁰-GARSG) | | 35 min |
| 33 | Api 300 (tPEG⁷⁵⁰-GARSG) | | 45 min |
| 36 | Api 300 (tPEG⁵⁰⁰⁰-GARSG) | | 69% (6 h)** |
| 31 | Api 300 (sPEG⁷⁵⁰-GAARSG) | | 45 min |
| 34 | Api 300 (tPEG⁷⁵⁰-GAARSG) | | 35 min |
| 37 | Api 300 (tPEG⁵⁰⁰⁰-GAARSG) | | 76% (6 h)** |
| 38 | Api 301 (SG)⁺ | | 60 % (4 h)** |
| 42 | Api 301 (sPEG⁷⁵⁰-GRSG) | | 30 min |
| 45 | Api 301 (tPEG⁷⁵⁰-GRSG) | | 55 min |
| 48 | Api 301 (tPEG⁵⁰⁰⁰-GRSG) | | 75% (6 h)** |
| 43 | Api 301 (sPEG⁷⁵⁰-GARSG) | | 45 min |
| 46 | Api 301 (tPEG⁷⁵⁰-GARSG) | | 45 min |
| 49 | Api 301 (tPEG⁵⁰⁰⁰-GARSG) | | 62% (6 h)** |
| 44 | Api 301 (sPEG⁷⁵⁰-GAARSG) | | 55 min |
| 47 | Api 301 (tPEG⁷⁵⁰-GAARSG) | | 50 min |
| 50 | Api 301 (tPEG⁵⁰⁰⁰-GAARSG) | | 78% (5 h)** |
| 51 | GAR-Onc 72* | | 85% (4 h)** |
| 52 | GAR-Onc 110* | | 75% (4 h)** |
| 55 | sPEG750-GAR-Onc 72 | | 6h |
| 56 | sPEG750-GAR-Onc 110 | | 73% (6 h)** |
| 57 | tPEG750-GAR-Onc 72 | | 55% (6 h)** |
| 58 | tPEG750-GAR-Onc 110 | | 64% (6 h)** |
| 59 | tPEG⁵⁰⁰⁰-GAR-Onc 72 | | 86% (6 h)** |
| 60 | tPEG⁵⁰⁰⁰-GAR-Onc 110 | | 96% (6 h)** |

| | | | |
|---|---|---|---|
| mit * sind Vergleichsbeispiele markiert, mit + sind Spaltungsprodukte markiert. **Rest ungespaltenes Peptid nach angegebener Zeit (statt Halbwertszeit) | | | |

Die Halbwertszeiten (bzw. Rest ungespaltenes Peptid nach angegebener Zeit) beziehen sich bei den freien Peptiden (Api 137, Api300 (SG) und Api 301 (SG)) auf die Spaltstelle im Peptide (nach Arg-16). Bei den restlichen Derivaten beziehen sich die Werte auf die Spaltung im Linker in der Seitenkette unter Freisetzung von Api 137 (SG), Api300 (SG) und Api 301 (SG) bzw. von Onc 72 und Onc 110.

In der Erfindungsbeschreibung werden folgende Abkürzungen verwendet:
- Agp: 2-Amino-3 -guanidinopropionsäure
- BOC: *tert*.-Butyloxy-carbonyl
- ^{t}Bu: *tert*.-Butylether
- Dap: 2,3-Diaminopropionsäure
- DCC: Dicyclohexylcarbodiimid
- DCM: Dichlormethan
- DIC: Diisopropylcarbodiimid
- DMF: Dimethylformamid
- *E. coli*: *Escherichia coli*
- *eq.*: *equivalents per mol,* Moläquivalente
- Fmoc: Fluorenylmethoxycarbonyl
- Guan: Guanidino-Gruppe (am N-Terminus)
- Hyp: *trans*-4-Hydroxyprolin
- HBTU: 2-(1H-Benzotriazol-1-yl)-1,1,3,3-tetramethyluronium-hexafluorophosphat
- HOBt: 1-Hydroxybenzotriazol
- LC-MS: Flüssigchromatographie (engl. *Liquid Chromatography*) mit Massenspektrometrie-Kopplung
- MALDI-TOF: Matrix unterstützte Laser-Desorption/Ionisierung (engl. *Matrix Assisted Laser Desorption*/*Ionization*) mit Flugzeitanalyse (engl. *time of flight*)
- MHK: minimalen Hemmkonzentration
- MS: Massenspektrometrie (engl. *Mass Spectrometry)*
- Mtt: 4-Methyltrityl
- NHS: *N*-Hydroxysuccinimid
- NMM: *N*-Methylmorpholine
- O: Ornithin
- O^{t}Bu: *tert*.-Butylester
- PBS: Phosphat-gepufferte Salzlösung (engl. *Phospho-buffered Saline)*
- PEG: Polyethylenglykol
- sPEG: über Säuregruppe verknüpftes PEG
- tPEG: über Thiolgruppe verknüpftes PEG
- Pra: Nα-(9-Fluorenylmethyloxycarbonyl)-L-progargylglycine
- RP-HPLC: Umkehrphasen-Hochleistungsflüssigkeitschromatographie (engl. *reversed phase high performance liquid chromatography)*
- RT: Raumtemperatur
- TCA: Trichloressigsäure
- TFA: Trifluoressigsäure
- Tle: L-tertiär-Leucine (L-tertiär -Butylglycine)
- Tris: Tris(hydroxymethyl)-aminomethan
- TSB: *Tryptic Soy Broth,* tryptisches Soja Medium

## Patentansprüche

1. Modifiziertes Peptid, welches einer der folgenden Sequenzen enthält:
NT-X₁X₂NX₃PVYIPX₄X₅RPPHP-CT (Formel 1)
NT-X₁NX₂X₃PVYIPX₄X₅RPPHP-CT (Formel 2)
NT-X₂NNX₃PVYIPX₄X₅RPPHP-CT (Formel 3)
wobei **X₁** ein Aminosäurerest ist, dessen Seitenkette unter physiologischen Bedingungen positiv geladen ist, bevorzugt O,
wobei **X₂** ein Aminosäurerest mit einer Aminogruppe in der Seitenkette ist,
wobei **X₃** ein Aminosäurerest ist, dessen Seitenkette unter physiologischen Bedingungen positiv geladen ist, bevorzugt R,
wobei **X₄** ein Aminosäurerest ist, dessen Seitenkette unter physiologischen Bedingungen positiv geladen ist, bevorzugt R,
wobei **X₅** Prolin oder ein Prolinderivat ist,
wobei CT der C-Terminus ist oder ein Peptid mit 1 bis 4 Aminosäureresten, bevorzugt ist ein Dipetid mit der Sequenz RL,
^{w}obei NT der N-Terminus ist, der bevorzugt guanidiert ist,
**dadurch gekennzeichnet, dass** an eine Aminogruppe in der Seitenkette von **X₁** oder **X₂** über einen Peptidlinker eine lineare oder verzweigte Polyethylenglykol-Polymerkette gebunden ist, wobei der Linker aus folgender Formel besteht:
G(L)ₖRSG
mit L ausgewählt aus Alanin, Glycin und Serin und k ausgewählt ist aus ganzen Zahlen von 1 bis 6,
**dadurch gekennzeichnet, dass** die Polyethylenglykol-Polymerkette ein Molekulargewicht von 500 bis 40000 Da aufweist,
wobei die Polyethylenglykol-Polymerkette an die alpha-Aminogruppe des ersten Aminosäurerests des Linkers gebunden ist.

2. Modifiziertes Peptid nach Anspruch 1, **dadurch gekennzeichnet, dass** X₁ und/oder X₂ ein L-Ornithinrest ist.

3. Modifiziertes Peptid nach einem der Ansprüche 1 bis 2, welches eine der folgenden Sequenzen enthält:
ONORPVYIPRPRPPHPRL (SEQ ID No. 9),
OONRPVYIPRPRPPHPRL (SEQ ID No. 10) oder
ONNRPVYIPRPRPPHPRL (SEQ ID No. 11) mit O = L-Ornithin,
wobei die alpha-Aminogruppe des ersten Ornithins guanidiert ist und an die delta-Aminogruppe des ersten oder zweiten Ornithins über einen Peptidlinker, bevorzugt ausgewählt aus GARSG, GAARSG, GAAARSG und GAAAARSG eine lineare oder verzweigte Polyethylenglykol-Polymerkette gebunden ist.

4. Modifiziertes Peptid, welches eine der folgenden Sequenzen enthält:
NT-X₁-D2-K3-P4-P5-Y6-L7-P8-R9-P10-X₂-P12-P13-R14-X₃-I16-Y17-N18-X₄-CT
NT-X₁-D2-K3-P4-P5-Y6-L7-P8-R9-P10-X₂-P12-P13-R14-X₃-I16-Y17-N18-N19-X₄-CT
NT-X₁-D2-K3-P4-P5-Y6-L7-P8-R9-P10-X₂-P12-P13-R14-X₃-I16-P17-N18-X₄-CT
X₁ ist ein Rest mit einer unpolaren, hydrophoben Seitenkette oder ein Aminosäurerest, dessen Seitenkette unter physiologischen Bedingungen positiv geladen ist oder eine positiven Nettoladung besitzt;
D₂ ist ein Asparaginsäure- oder Glutaminsäurerest,
K₃ ist ein Rest mit einer positiven Nettoladung oder einer unter physiologischen Bedingungen positiv geladenen Seitenkette, bevorzugt Lysin oder Arginin,
X₂ und X₄ sind unabhängig von einander ausgewählt aus Resten mit einer positiven Nettoladung oder einer unter physiologischen Bedingungen positiv geladenen Seitenkette;
X₃ ist ein Rest mit einer positiven Nettoladung oder einer unter physiologischen Bedingungen positiv geladenen Seitenkette oder Prolin oder ein Prolinderivat;
L₇ und I₁₆ sind unabhängig voneinander ausgewählt aus Resten mit einer unpolaren, hydrophoben Seitenkette, bevorzugt Leucin, Isoleucin und Valin,
Y₆ und Y₁₇ sind jeweils Tyrosin, R₉ und R₁₄ sind jeweils Arginin, N₁₈ und N₁₉ sind jeweils Asparagin oder Glutamin, P₄, P₅, P₈, P₁₀, P₁₂, P₁₃ und P₁₇ sind unabhängig voneinander ausgewählt aus Prolin und Prolinderivaten oder Hydroxyprolin und Hydroxyprolinderivaten,
wobei gegebenenfalls P₁₃ und R₁₄ vertauscht sind, und/oder
gegebenenfalls ein oder zwei der Reste ausgewählt aus D₂, P₄, P₅, P₈, P₁₀, P₁₂ P₁₃, P₁₇ und Y₁₇ durch einen beliebigen Rest ersetzt sind,
NT ist der modifizierte N-Terminus der Aminosäure X₁,
CT ist der freie oder modifizierte C-Terminus der Aminosäure X₄,
**dadurch gekennzeichnet, dass** an NT über einen Peptidlinker eine lineare oder verzweigte Polyethylenglykol-Polymerkette gebunden ist und
wobei in den Peptidlinker 3 bis 8 Aminosäurereste eine tryptische Schnittstelle unmittelbar zwischen dem Polymer und dem antimikrobiellen Peptid eingefügt ist und der Linker die folgende Formel:
G(L)ₖR
mit L ausgewählt aus Alanin, Glycin und Serin und k ausgewählt aus 1, 2 oder 3 hat,
**dadurch gekennzeichnet, dass** die Polyethylenglykol-Polymerkette ein Molekulargewicht von 500 bis 40000 Da aufweist,
wobei die Polyethylenglykol-Polymerkette an die alpha-Aminogruppe des ersten Aminosäurerests des Linkers gebunden ist,
wobei die Kopplung der Polyethylenglykol-Polymerkette an den Peptidlinker entweder direkt oder mittels eines kurzen organischen Linkers und/oder durch Ausbildung einer Thioetherbindung erfolgt.

5. Modifiziertes Peptid nach Anspruch 4, welches eine der folgenden Sequenzen enthält:
VDKPPYLPRPRPPROIYNO-NH₂ (SEQ ID No. 12) oder
VDKPPYLPRPRPHypRHypTleYNO-NH₂ (SEQ ID No. 13) mit 0 = L-Ornithin,
Hyp = L-4-Hydroxyprolin,
Tle = L-t-Leucine (L-t-Butylglycine),
wobei an die N-terminale Aminogruppe (alpha-Aminogruppe des ersten Aminosäurerests V) über den Peptidlinker eine lineare oder verzweigte Polyethylenglykol-Polymerkette gebunden ist.

6. Ein modifiziertes Peptid gemäß einem der Ansprüche 1 bis 5 zur Verwendung in der Medizin.

7. Modifiziertes Peptid nach einem der Ansprüche 1 bis 5 zur Verwendung als Antibiotikum.

8. Verwendung eines modifizierten Peptids nach einem der Ansprüche 1 bis 5 in einem Desinfektions- oder Reinigungsmittel, als Konservierungsmittel oder in einem Verpackungsmaterial oder zur Behandlung von Verunreinigungen mit Mikroben, Bakterien oder Pilzen.

9. Modifiziertes Peptid nach einem der Ansprüche 1 bis 5 zur Verwendung zur Behandlung von mikrobiellen, bakteriellen oder Pilz-Infektionen.

## Claims

1. Modified peptide which contains one of the following sequences:
NT-X₁X₂NX₃PVYIPX₄X₅RPPHP-CT (formula 1)
NT-X₁NX₂X₃PVYIPX₄X₅RPPHP-CT (formula 2)
NT-X₂NNX₃PVYIPX₄X₅RPPHP-CT (formula 3)
where X₁ is an amino acid residue, the side chain of which is positively charged under physiological conditions, preferably O,
where X₂ is an amino acid residue having an amino group in the side chain,
where X₃ is an amino acid residue, the side chain of which is positively charged under physiological conditions, preferably R,
where X₄ is an amino acid residue, the side chain of which is positively charged under physiological conditions, preferably R,
where X₅ is proline or a proline derivative,
where CT is the C-terminus or a peptide having 1 to 4 amino acid residues, preferably a dipeptide having the sequence RL,
where NT is the N-terminus, which is preferably guanidised,
**characterised in that** a linear or branched polyethylene glycol polymer chain is bonded to an amino group in the side chain of X₁ or X₂ via a peptide linker,
the linker consisting of the following formula:
G(L)ₖRSG
where L is selected from alanine, glycine and serine and k is selected from whole numbers of between 1 and 6,
**characterised in that** the polyethylene glycol polymer chain has a molecular weight of from 500 to 40000 Da,
the polyethylene glycol polymer chain being bonded to the alpha amino group of the first amino acid residue of the linker.

2. Modified peptide according to claim 1, **characterised in that** X₁ and/or X₂ is an L-ornithine residue.

3. Modified peptide according to either claim 1 or claim 2, which peptide contains one of the following of the sequences:
ONORPVYIPRPRPPHPRL (SEQ ID No. 9),
OONRPVYIPRPRPPHPRL (SEQ ID No. 10) or
ONNRPVYIPRPRPPHPRL (SEQ IN No. 11) where O = L-ornithine,
wherein the alpha amino group of the first ornithine is guanidised, and a linear or branched polyethylene glycol polymer chain, preferably selected from GARSG, GAARSG, GAAARSG and GAAAARSG, is bonded to the delta amino group of the first or second ornithine via a peptide linker.

4. Modified peptide, which contains one of the following sequences:
NT-X₁-D2-K3-P4-P5-Y6-L7-P8-R9-P10-X₂-P12-P13-R14-X₃-I16-Y17-N18-X₄-CT
NT-X₁-D2-K3-P4-P5-Y6-L7-P8-R9-P10-X₂-P12-P13-R14-X₃-I16-Y17-N18-N19-X₄-CT
NT-X₁-D2-K3-P4-P5-Y6-L7-P8-R9-P10-X₂-P12-P13-R14-X₃-I16-P17-N18-X₄-CT
X₁ is a residue having a non polar, hydrophobic side chain, or an amino acid residue, the side chain of which is positively charged or has a positive net charge under physiological conditions;
D₂ is an aspartic acid or glutamic acid residue;
K₃ is a residue having a positive net charge or a side chain that is positively charged under physiological conditions, preferably lysine or arginine;
X₂ and X₄ are, independently of one another, selected from residues having a positive net charge or a side chain that is positively charged under physiological conditions;
X₃ is a residue having a positive net charge or a side chain that is positively charged under physiological conditions, or proline or a proline derivative;
L₇ and I₁₆ are, independently of one another, selected from residues having a non polar, hydrophobic side chain, preferably leucine, isoleucine and valine;
Y₆ and Y₁₇ are in each case tyrosine; R₉ and R₁₄ are in each case arginine; N₁₈ and N₁₉ are in each case asparagine or glutamine, and P₄, P₅, P₈, P₁₀, P₁₂, P₁₃ and P₁₇ are, independently of one another, selected from proline and proline derivatives or hydroxyproline and hydroxyproline derivatives, P₁₃ and R₁₄ being optionally exchanged, and/or one or two of the residues selected from D₂, P₄, P₅, P₈, P₁₀, P₁₂, P₁₃, P₁₇ and Y₁₇ being optionally replaced by any residue;
NT is the modified N-terminus of the amino acid X₁;
CT is the free or modified C-terminus of the amino acid X₄,
**characterised in that** a linear or branched polyethylene glycol polymer chain is bonded to NT via a peptide linker, and a tryptic cleavage site being inserted directly between the polymer and the antimicrobial peptide in the peptide linker, the linker having the following formula:
G(L)ₖR
where L is selected from alanine, glycine and serine, and k is selected from 1, 2 or 3,
**characterised in that** the polyethylene glycol polymer chain has a molecular weight of from 500 to 40000 Da,
the polyethylene glycol polymer chain being bonded to the alpha amino group of the first amino acid residue of the linker,
the coupling of the polyethylene glycol polymer chain to the peptide linker taking place either directly or by means of a short organic linker and/or by formation of a thioether bond.

5. Modified peptide according to claim 4, which peptide contains one of the following sequences:
VDKPPYLPRPRPPROIYNO-NH₂ (SEQ ID No. 12) or
VDKPPYLPRPRPHypRHypTIeYNO-NH₂ (SEQ ID No. 13) where O = L-ornithine,
Hyp = L-4-hydroxyproline, Tie = L-t-leucine (L-t-butyl glycine),
wherein a linear or branched polyethylene glycol polymer chain is bonded to the N-terminal amino group (alpha amino group of the first amino residue V) via the peptide linker.

6. Modified peptide according to any of claims 1 to 5 for use in medicine.

7. Modified peptide according to any of claims 1 to 5 for use as an antibiotic.

8. Use of a modified peptide according to any of claims 1 to 5 in a disinfection agent, cleaning agent or in a packaging material or for the treatment of contamination by microbes, bacteria or fungi.

9. Modified peptide according to any of claims 1 to 5 for the treatment of microbial, bacterial or fungal infections.

## Revendications

1. Peptide modifié, lequel contient l'une des séquences suivantes :
NT-X₁X₂NX₃PVYIPX₄X₅RPPHP-CT (formule 1)
NT-X₁NX₂X₃PVYIPX₄X₅RPPHP-CT (formule 2)
NT-X₂NNX₃PVYIPX₄X₅RPPHP-CT (formule 3)
où **X₁** est un résidu d'acide aminé, dont la chaîne latérale est chargée positivement dans des conditions physiologiques, de préférence O,
où **X**₂ est un résidu d'acide aminé comportant un groupe amino dans la chaîne latérale,
où **X**₃ est un résidu d'acide aminé, dont la chaîne latérale est chargée positivement dans des conditions physiologiques, de préférence R,
où **X**₄ est un résidu d'acide aminé, dont la chaîne latérale est chargée positivement dans des conditions physiologiques, de préférence R,
où **X**₅ est de la proline ou un dérivé de la proline,
où CT est l'extrémité C-terminale ou un peptide comportant 1 à 4 résidus d'acides aminés, est de préférence un dipeptide avec la séquence RL,
où NT est l'extrémité N-terminale, qui est de préférence guanidinée,
**caractérisé en ce qu'**une chaîne polymère de polyéthylène glycol linéaire ou ramifiée est liée à un groupe amino dans la chaîne latérale de **X₁** ou **X₂** via un lieur peptidique,
le lieur étant composé de la formule suivante :
G(L)ₖRSG
L étant sélectionné parmi l'alanine, la glycine et la sérine, et k étant sélectionné parmi des nombres entiers allant de 1 à 6,
**caractérisé en ce que** la chaîne polymère de polyéthylène glycol a une masse molaire de 500 à 40 000 Da,
la chaîne polymère de polyéthylène glycol étant liée au groupe alpha-amino du premier résidu d'acide aminé du lieur.

2. Peptide modifié selon la revendication 1, **caractérisé en ce que** X₁ et/ou X₂ est un résidu de L-ornithine.

3. Peptide modifié selon l'une des revendications 1 et 2, lequel contient l'une des séquences suivantes :
ONORPVYIPRPRPPHPRL (SEQ ID No. 9),
OONRPVYIPRPRPPHPRL (SEQ ID No. 10) ou
ONNRPVYIPRPRPPHPRL (SEQ ID No. 11) selon lesquelles O = L-ornithine,
le groupe alpha-amino de la première ornithine est guanidiné et une chaîne polymère de polyéthylène glycol linéaire ou ramifiée étant liée au groupe delta-amino de la première ou deuxième ornithine via un lieur peptidique, de préférence sélectionné parmi GARSG, GAARSG, GAAARSG et GAAAARSG.

4. Peptide modifié, lequel contient l'une des séquences suivantes :
NT-X₁-D2-K3-P4-P5-Y6-L7-P8-R9-P10-X₂-P12-P13-R14-X₃-I16-Y17-N18-X₄-CT
NT-X₁-D2-K3-P4-P5-Y6-L7-P8-R9-P10-X₂-P12-P13-R14-X₃-I16-Y17-N18-N19-X₄-CT
NT-X₁-D2-K3-P4-P5-Y6-L7-P8-R9-P10-X₂-P12-P13-R14-X₃-I16-P17-N18-X₄-CT
X₁ est un résidu d'acide aminé comportant une chaîne latérale hydrophobe non polaire ou un résidu d'acide aminé, dont la chaîne latérale est chargée positivement dans des conditions physiologiques ou possède une charge nette positive ;
D₂ est un résidu d'acide asparaginique ou d'acide glutamique,
K₃ est un résidu ayant une charge nette positive ou une chaîne latérale chargée positivement dans des conditions physiologiques, de préférence la lysine ou l'arginine,
X₂ et X₄ sont sélectionnés indépendamment l'un de l'autre à partir de résidus ayant une charge nette positive ou une chaîne latérale chargée positivement dans des conditions physiologiques ;
X₃ est un résidu ayant une charge nette positive ou une chaîne latérale chargée positivement dans des conditions physiologiques ou de la proline ou un dérivé de la proline ;
L₇ et I₁₆ sont sélectionnés indépendamment l'un de l'autre à partir de résidus comportant une chaîne latérale hydrophobe non polaire, de préférence de la leucine, de l'isoleucine et de la valine,
Y₆ et Y₁₇ sont chacun de la tyrosine, R₉ et R₁₄ sont chacun de l'arginine, N₁₈ et N₁₉ sont chacun de l'asparagine ou de la glutamine, P₄, P₅, P₈, P₁₀, P₁₂, P₁₃ et P₁₇ sont sélectionnés chacun indépendamment les uns des autres parmi la proline et des dérivés de la proline ou l'hydroxyproline et des dérivés de l'hydroxyproline, P₁₃ et R₁₄ étant échangés le cas échéant,
et/ou un ou deux des résidus sélectionnés parmi D₂, P₄, P₅, P₈, P₁₀, P₁₂, P₁₃, P₁₇ et Y₁₇ étant remplacés par un résidu quelconque,
NT est l'extrémité N-terminale modifiée de l'acide aminé X₁,
CT est l'extrémité C-terminale libre ou modifiée de l'acide aminé X₄,
**caractérisé en ce qu'**une chaîne polymère de polyéthylène glycol linéaire ou ramifiée est liée à NT via un lieur peptidique, une site de clivage tryptique étant insérée directement entre le polymère et le peptide antimicrobien dans le lieur peptidique ayant la formule suivante :
G(L)ₖR
L étant sélectionné parmi l'alanine, la glycine et la sérine et k étant sélectionné parmi les chiffres 1, 2 ou 3, **caractérisé en ce que** la chaîne polymère de polyéthylène glycol possède une masse molaire de 500 à 40 000 Da,
la chaîne polymère de polyéthylène glycol étant liée au groupe alpha-amino du premier résidu d'acide aminé du lieur,
le couplage de la chaîne polymère de polyéthylène glycol au lieur peptidique étant effectué soit directement soit au moyen d'un lieur organique court et/ou par la formation d'une liaison de thioéther.

5. Peptide modifié selon la revendication 4, lequel contient l'une des séquences suivantes :
VDKPPYLPRPRPPROIYNO-NH₂ (SEQ ID No. 12) ou
VDKPPYLPRPRPHypRHypTIeYNO-NH₂ (SEQ ID No. 13) selon lesquelles O = L-ornithine, Hyp = L-4-hydroxyproline, Tle = L-t-leucine (L-t-butylglycol),
une chaîne polymère de polyéthylène glycol linéaire ou ramifiée étant liée au groupe amino N-terminal (groupe alpha-amino du premier résidu d'acide aminé V) via le lieur peptidique.

6. Peptide modifié selon l'une des revendications 1 à 5 pour utilisation médicale.

7. Peptide modifié selon l'une des revendications 1 à 5 pour utilisation en tant qu'antibiotique.

8. Utilisation d'un peptide modifié selon l'une des revendications 1 à 5 dans un agent désinfectant ou nettoyant ou dans un matériau d'emballage ou pour le traitement d'impuretés comportant des microbes, des bactéries ou des champignons.

9. Peptide modifié selon l'une des revendications 1 à 5 pour le traitement d'infections microbiennes ou bactériennes, ou de mycoses.


### SEQUENCE LISTING / SEQUENZPROTOKOLL

<110> Universität Leipzig
<120> Modifizierte antibiotische Peptide mit variabler systemischer Freisetzung
<130> 00401P0054DEWO
<150> DE 10 2011 118 029.3
   <151> 20.06.2011
<160> 66
<170> BiSSAP 1.0
<210> 1
   <211> 18
   <212> PRT
   <213> Apis mellifera
<400> 1
   Gly Asn Asn Arg Pro Val Tyr Ile Pro Gln Pro Arg Pro Pro His Pro
   1               5                   10                  15
   Arg Ile
<210> 2
   <211> 18
   <212> PRT
   <213> Apis mellifera
<400> 2
   Gly Asn Asn Arg Pro Val Tyr Ile Pro Gln Pro Arg Pro Pro His Pro
   1               5                   10                  15
   Arg Leu
<210> 3
   <211> 19
   <212> PRT
   <213> Drosophila melanogaster
<400> 3
   Gly Lys Pro Arg Pro Tyr Ser Pro Arg Pro Thr Ser His Pro Arg Pro
   1               5                   10                  15
   Ile Arg Val
<210> 4
   <211> 16
   <212> PRT
   <213> Myrmecia gulosa
<400> 4
   Gly Arg Pro Asn Pro Val Asn Asn Lys Pro Thr Pro Tyr Pro His Leu
   1               5                   10                  15
<210> 5
   <211> 20
   <212> PRT
   <213> Pyrrhocoris apterus
<220>
   <221> misc_feature
   <222> (20)..(20)
   <223> Xaa is Asparagine amide
<400> 5
   Val Asp Lys Gly Ser Tyr Leu Pro Arg Pro Thr Pro Pro Arg Pro Ile
   1               5                   10                  15
   Tyr Asn Arg Xaa
               20
<210> 6
   <211> 15
   <212> PRT
   <213> Palomena prasina
<400> 6
   Val Asp Lys Pro Asp Tyr Arg Pro Arg Pro Arg Pro Pro Asn Met
   1               5                   10                  15
<210> 7
   <211> 34
   <212> PRT
   <213> Oncopeltus fasciatus
<400> 7
   Glu Val Ser Leu Lys Gly Glu Gly Gly Ser Asn Lys Gly Phe Ile Gln
   1               5                   10                  15
   Gly Ser Gly Thr Lys Thr Leu Phe Gln Asp Asp Lys Thr Lys Leu Asp
               20                  25                  30
   Gly Thr
<210> 8
   <211> 20
   <212> PRT
   <213> Oncopeltus fasciatus
<220>
   <221> misc_feature
   <222> (11)..(11)
   <223> Xaa is Pro or any other naturally occurring amino acid
<220>
   <221> misc_feature
   <222> (19)..(20)
   <223> Optional residues
<400> 8
   Val Asp Lys Pro Pro Tyr Leu Pro Arg Pro Xaa Pro Pro Arg Arg Ile
   1               5                   10                  15
   Tyr Asn Asn Arg
<210> 9
   <211> 18
   <212> PRT
   <213> Artificial Sequence
<220>
   <223> Artificial Peptide
<220>
   <221> misc_feature
   <222> (1)..(1)
   <223> Xaa is Ornithine
<220>
   <221> misc_feature
   <222> (3)..(3)
   <223> Xaa is Ornithine
<400> 9
   Xaa Asn Xaa Arg Pro Val Tyr Ile Pro Arg Pro Arg Pro Pro His Pro
   1               5                   10                  15
   Arg Leu
<210> 10
   <211> 18
   <212> PRT
   <213> Artificial Sequence
<220>
   <223> Artificial Peptide
<220>
   <221> misc_feature
   <222> (1)..(1)
   <223> Xaa is Ornithine
<220>
   <221> misc_feature
   <222> (2)..(2)
   <223> Xaa is Ornithine
<400> 10
   Xaa Xaa Asn Arg Pro Val Tyr Ile Pro Arg Pro Arg Pro Pro His Pro
   1               5                   10                  15
   Arg Leu
<210> 11
   <211> 18
   <212> PRT
   <213> Artificial Sequence
<220>
   <223> Artificial Peptide
<220>
   <221> misc_feature
   <222> (1)..(1)
   <223> Xaa is Ornithine
<400> 11
   Xaa Asn Asn Arg Pro Val Tyr Ile Pro Arg Pro Arg Pro Pro His Pro
   1               5                   10                  15
   Arg Leu
<210> 12
   <211> 19
   <212> PRT
   <213> Artificial Sequence
<220>
   <223> Artificial Peptide
<220>
   <221> misc_feature
   <222> (15)..(15)
   <223> Xaa is Ornithine
<220>
   <221> misc_feature
   <222> (19)..(19)
   <223> Xaa is Ornithine amide
<400> 12
   Val Asp Lys Pro Pro Tyr Leu Pro Arg Pro Arg Pro Pro Arg Xaa Ile
   1               5                   10                  15
   Tyr Asn Xaa
<210> 13
   <211> 19
   <212> PRT
   <213> Artificial Sequence
<220>
   <223> Artificial Peptide
<220>
   <221> misc_feature
   <222> (13)..(13)
   <223> Xaa is 4-Hydroxyproline
<220>
   <221> misc_feature
   <222> (15)..(15)
   <223> Xaa is 4-Hydroxyproline
<220>
   <221> misc_feature
   <222> (16)..(16)
   <223> Xaa is tert-Leucine
<220>
   <221> misc_feature
   <222> (19)..(19)
   <223> Xaa is Ornithine amide
<400> 13
   Val Asp Lys Pro Pro Tyr Leu Pro Arg Pro Arg Pro Xaa Arg Xaa Xaa
   1               5                   10                  15
   Tyr Asn Xaa
<210> 14
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (18)..(18)
   <223> Xaa is Leucin amide
<400> 14
   Xaa Asn Asn Arg Pro Val Tyr Ile Pro Arg Pro Arg Pro Pro His Pro
   1               5                   10                  15
   Arg Xaa
<210> 15
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (17)..(17)
   <223> Xaa is Ornithine
<220>
   <221> misc_feature
   <222> (18)..(18)
   <223> Xaa is Leucin amide
<400> 15
   Xaa Asn Asn Arg Pro Val Tyr Ile Pro Arg Pro Arg Pro Pro His Pro
   1               5                   10                  15
   Xaa Xaa
<210> 16
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<400> 16
   Xaa Asn Asn Arg Pro Val Tyr Ile Pro Arg Pro Arg Pro Pro His Pro
   1               5                   10                  15
   Arg Leu
<210> 17
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Polyethylene glycole 750 linked directly to delta-NH2 of Ornithine
<400> 17
   Xaa Asn Asn Arg Pro Val Tyr Ile Pro Arg Pro Arg Pro Pro His Pro
   1               5                   10                  15
   Arg Leu
<210> 18
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Ser-Gly-Linker bound to delta-NH2 of Ornithine
<400> 18
   Xaa Asn Asn Arg Pro Val Tyr Ile Pro Arg Pro Arg Pro Pro His Pro
   1               5                   10                  15
   Arg Leu
<210> 19
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Gly-Arg-Ser-Gly-Linker bound to delta-NH2 of Ornithine
<400> 19
   Xaa Asn Asn Arg Pro Val Tyr Ile Pro Arg Pro Arg Pro Pro His Pro
   1               5                   10                  15
   Arg Leu
<210> 20
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Gly-Ala-Arg-Ser-Gly-Linker bound to delta-NH2 of Ornithine
<400> 20
   Xaa Asn Asn Arg Pro Val Tyr Ile Pro Arg Pro Arg Pro Pro His Pro
   1               5                   10                  15
   Arg Leu
<210> 21
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Polyethylene glycole 750 bound directly to N-terminus of Gly-Arg-Ser-Gly-Linker bound to delta-NH2 of Ornithine
<400> 21
   Xaa Asn Asn Arg Pro Val Tyr Ile Pro Arg Pro Arg Pro Pro His Pro
   1               5                   10                  15
   Arg Leu
<210> 22
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Polyethylene glycole 750 bound directly to N-terminus of Gly-Ala-Arg-Ser-Gly-Linker bound to delta-NH2 of Ornithine
<400> 22
   Xaa Asn Asn Arg Pro Val Tyr Ile Pro Arg Pro Arg Pro Pro His Pro
   1               5                   10                  15
   Arg Leu
<210> 23
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Polyethylene glycole 5000 bound directly to N-terminus of Gly-Ala-Arg-Ser-Gly-Linker bound to delta-NH2 of Ornithine
<400> 23
   Xaa Asn Asn Arg Pro Val Tyr Ile Pro Arg Pro Arg Pro Pro His Pro
   1               5                   10                  15
   Arg Leu
<210> 24
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine with Polyethylene glycole 20000 bound to N-terminus of Gly-Ala-Arg-Ser-Gly-Linker bound to delta-NH2 of Ornithine
<400> 24
   Xaa Asn Asn Arg Pro Val Tyr Ile Pro Arg Pro Arg Pro Pro His Pro
   1               5                   10                  15
   Arg Leu
<210> 25
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with ser-Gly-Linker bound to delta-NH2 of Ornithine
<400> 25
   Xaa Xaa Asn Arg Pro Val Tyr Ile Pro Arg Pro Arg Pro Pro His Pro
   1               5                   10                  15
   Arg Leu
<210> 26
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with Gly-Arg-Ser-Gly-Linker bound to delta-NH2 of Ornithine
<400> 26
   Xaa Xaa Asn Arg Pro Val Tyr Ile Pro Arg Pro Arg Pro Pro His Pro
   1               5                   10                  15
   Arg Leu
<210> 27
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with Gly-Ala-Arg-Ser-Gly-Linker bound to delta-NH2 of Ornithine
<400> 27
   Xaa Xaa Asn Arg Pro Val Tyr Ile Pro Arg Pro Arg Pro Pro His Pro
   1               5                   10                  15
   Arg Leu
<210> 28
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with Gly-Ala-Ala-Arg-Ser-Gly-Linker bound to delta-NH2 of Ornithine
<400> 28
   Xaa Xaa Asn Arg Pro Val Tyr Ile Pro Arg Pro Arg Pro Pro His Pro
   1               5                   10                  15
   Arg Leu
<210> 29
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with Polyethylene glycole 750 bound to N-terminus of
   Gly-Arg-Ser-Gly-Linker bound to delta-NH2 of Ornithine
<400> 29
   Xaa Xaa Asn Arg Pro Val Tyr Ile Pro Arg Pro Arg Pro Pro His Pro
   1               5                   10                  15
   Arg Leu
<210> 30
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with Polyethylene glycole 750 bound to N-terminus of
   Gly-Ala-Arg-Ser-Gly-Linker bound to delta-NH2 of Ornithine
<400> 30
   Xaa Xaa Asn Arg Pro Val Tyr Ile Pro Arg Pro Arg Pro Pro His Pro
   1               5                   10                  15
   Arg Leu
<210> 31
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with Polyethylene glycole 750 bound to N-terminus of
   Gly-Ala-Ala-Arg-Ser-Gly-Linker bound to delta-NH2 of Ornithine
<400> 31
   Xaa Xaa Asn Arg Pro Val Tyr Ile Pro Arg Pro Arg Pro Pro His Pro
   1               5                   10                  15
   Arg Leu
<210> 32
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with thiol modified Polyethylene glycole 750 bound to iodoacetylated N-terminus of Gly-Arg-Ser-Gly-Linker bound to delta-NH2 of Ornithine
<400> 32
   Xaa Xaa Asn Arg Pro Val Tyr Ile Pro Arg Pro Arg Pro Pro His Pro
   1               5                   10                  15
   Arg Leu
<210> 33
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with thiol modified Polyethylene glycole 750 bound to iodoacetylated N-terminus of Gly-Ala-Arg-Ser-Gly-Linker bound to delta-NH2 of Ornithine
<400> 33
   Xaa Xaa Asn Arg Pro Val Tyr Ile Pro Arg Pro Arg Pro Pro His Pro
   1               5                   10                  15
   Arg Leu
<210> 34
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with thiol modified Polyethylene glycole 750 bound to iodoacetylated N-terminus of Gly-Ala-Ala-Arg-Ser-Gly-Linker bound to delta-NH2 of Ornithine
<400> 34
   Xaa Xaa Asn Arg Pro Val Tyr Ile Pro Arg Pro Arg Pro Pro His Pro
   1               5                   10                  15
   Arg Leu
<210> 35
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with thiol modified Polyethylene glycole 5000 bound to iodoacetylated N-terminus of Gly-Arg-Ser-Gly-Linker bound to delta-NH2 of Ornithine
<400> 35
   Xaa Xaa Asn Arg Pro Val Tyr Ile Pro Arg Pro Arg Pro Pro His Pro
   1               5                   10                  15
   Arg Leu
<210> 36
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with thiol modified Polyethylene glycole 5000 bound to iodoacetylated N-terminus of Gly-Ala-Arg-Ser-Gly-Linker bound to delta-NH2 of Ornithine
<400> 36
   Xaa Xaa Asn Arg Pro Val Tyr Ile Pro Arg Pro Arg Pro Pro His Pro
   1               5                   10                  15
   Arg Leu
<210> 37
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (2)..(2)
   <223> Xaa is Ornithine with thiol modified Polyethylene glycole 5000 bound to iodoacetylated N-terminus of Gly-Ala-Ala-Arg-Ser-Gly-Linker bound to delta-NH2 of Ornithine
<400> 37
   Xaa Xaa Asn Arg Pro Val Tyr Ile Pro Arg Pro Arg Pro Pro His Pro
   1               5                   10                  15
   Arg Leu
<210> 38
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Ser-Gly-Linker bound to delta-NH2 of Ornithine
<400> 38
   Xaa Asn Xaa Arg Pro Val Tyr Ile Pro Arg Pro Arg Pro Pro His Pro
   1               5                   10                  15
   Arg Leu
<210> 39
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Gly-Arg-Ser-Gly-Linker bound to delta-NH2 of Ornithine
<400> 39
   Xaa Asn Xaa Arg Pro Val Tyr Ile Pro Arg Pro Arg Pro Pro His Pro
   1               5                   10                  15
   Arg Leu
<210> 40
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Gly-Ala-Arg-Ser-Gly-Linker bound to delta-NH2 of Ornithine
<400> 40
   Xaa Asn Xaa Arg Pro Val Tyr Ile Pro Arg Pro Arg Pro Pro His Pro
   1               5                   10                  15
   Arg Leu
<210> 41
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Gly-Ala-Ala-Arg-Ser-Gly-Linker bound to delta-NH2 of Ornithine
<400> 41
   Xaa Asn Xaa Arg Pro Val Tyr Ile Pro Arg Pro Arg Pro Pro His Pro
   1               5                   10                  15
   Arg Leu
<210> 42
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Polyethylene glycole 750 bound to N-terminus of Gly-Arg-Ser-Gly-Linker bound to delta-NH2 of Ornithine
<400> 42
   Xaa Asn Xaa Arg Pro Val Tyr Ile Pro Arg Pro Arg Pro Pro His Pro
   1               5                   10                  15
   Arg Leu
<210> 43
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Polyethylene glycole 750 bound to N-terminus of Gly-Ala-Arg-Ser-Gly-Linker bound to delta-NH2 of Ornithine
<400> 43
   Xaa Asn Xaa Arg Pro Val Tyr Ile Pro Arg Pro Arg Pro Pro His Pro
   1               5                   10                  15
   Arg Leu
<210> 44
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with Polyethylene glycole 750 bound to N-terminus of Gly-Ala-Ala-Arg-Ser-Gly-Linker bound to delta-NH2 of Ornithine
<400> 44
   Xaa Asn Xaa Arg Pro Val Tyr Ile Pro Arg Pro Arg Pro Pro His Pro
   1               5                   10                  15
   Arg Leu
<210> 45
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with with thiol modified Polyethylene glycole 750 bound to iodoacetylated N-terminus of Gly-Arg-Ser-Gly-Linker bound to delta-NH2 of Ornithine
<400> 45
   Xaa Asn Xaa Arg Pro Val Tyr Ile Pro Arg Pro Arg Pro Pro His Pro
   1               5                   10                  15
   Arg Leu
<210> 46
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with with thiol modified Polyethylene glycole 750 bound to iodoacetylated N-terminus of Gly-Ala-Arg-Ser-Gly-Linker bound to delta-NH2 of Ornithine
<400> 46
   Xaa Asn Xaa Arg Pro Val Tyr Ile Pro Arg Pro Arg Pro Pro His Pro
   1               5                   10                  15
   Arg Leu
<210> 47
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with with thiol modified Polyethylene glycole 750 bound to iodoacetylated N-terminus of Gly-Ala-Ala-Arg-Ser-Gly-Linker bound to delta-NH2 of Ornithine
<400> 47
   Xaa Asn Xaa Arg Pro Val Tyr Ile Pro Arg Pro Arg Pro Pro His Pro
   1               5                   10                  15
   Arg Leu
<210> 48
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with with thiol modified Polyethylene glycole 5000 bound to iodoacetylated N-terminus of Gly-Arg-Ser-Gly-Linker bound to delta-NH2 of Ornithine
<400> 48
   Xaa Asn Xaa Arg Pro Val Tyr Ile Pro Arg Pro Arg Pro Pro His Pro
   1               5                   10                  15
   Arg Leu
<210> 49
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with with thiol modified Polyethylene glycole 5000 bound to iodoacetylated N-terminus of Gly-Ala-Arg-Ser-Gly-Linker bound to delta-NH2 of Ornithine
<400> 49
   Xaa Asn Xaa Arg Pro Val Tyr Ile Pro Arg Pro Arg Pro Pro His Pro
   1               5                   10                  15
   Arg Leu
<210> 50
   <211> 18
   <212> PRT
   <213> Artificial Sequence
<220>
   <223> Artificial Peptide
<220>
   <221> misc_feature
   <222> (1)..(1)
   <223> Xaa is N-Guanido-Ornithine
<220>
   <221> misc_feature
   <222> (3)..(3)
   <223> Xaa is Ornithine with with thiol modified Polyethylene glycole 5000 bound to iodoacetylated N-terminus of Gly-Ala-Ala-Arg-Ser-Gly-Linker bound to delta-NH2 of Ornithine
<400> 50
   Xaa Asn Xaa Arg Pro Val Tyr Ile Pro Arg Pro Arg Pro Pro His Pro
   1               5                   10                  15
   Arg Leu
<210> 51
   <211> 19
   <212> PRT
   <213> Artificial Sequence
<220>
   <223> Artificial Peptide
<220>
   <221> misc_feature
   <222> (15)..(15)
   <223> Xaa is Ornithine
<220>
   <221> misc_feature
   <222> (19)..(19)
   <223> Xaa is Ornithine amide
<400> 51
   Val Asp Lys Pro Pro Tyr Leu Pro Arg Pro Arg Pro Pro Arg Xaa Ile
   1               5                   10                  15
   Tyr Asn Xaa
<210> 52
   <211> 19
   <212> PRT
   <213> Artificial Sequence
<220>
   <223> Artificial Peptide
<220>
   <221> misc_feature
   <222> (13)..(13)
   <223> Xaa is 4-Hydroxyproline
<220>
   <221> misc_feature
   <222> (15)..(15)
   <223> Xaa is 4-Hydroxyproline
<220>
   <221> misc_feature
   <222> (16)..(16)
   <223> Xaa is tert-Leucine
<220>
   <221> misc_feature
   <222> (19)..(19)
   <223> Xaa is Ornithine amide
<400> 52
   Val Asp Lys Pro Pro Tyr Leu Pro Arg Pro Arg Pro Xaa Arg Xaa Xaa
   1               5                   10                  15
   Tyr Asn Xaa
<210> 53
   <211> 22
   <212> PRT
   <213> Artificial Sequence
<220>
   <223> Artificial Peptide
<220>
   <221> misc_feature
   <222> (18)..(18)
   <223> Xaa is Ornithine
<220>
   <221> misc_feature
   <222> (22)..(22)
   <223> Xaa is Ornithine amide
<400> 53
   Gly Ala Arg Val Asp Lys Pro Pro Tyr Leu Pro Arg Pro Arg Pro Pro
   1               5                   10                  15
   Arg Xaa Ile Tyr Asn Xaa
               20
<210> 54
   <211> 22
   <212> PRT
   <213> Artificial Sequence
<220>
   <223> Artificial Peptide
<220>
   <221> misc_feature
   <222> (16)..(16)
   <223> Xaa is 4-Hydroxyproline
<220>
   <221> misc_feature
   <222> (18)..(18)
   <223> Xaa is 4-Hydroxyproline
<220>
   <221> misc_feature
   <222> (19)..(19)
   <223> Xaa is tert-Leucine
<220>
   <221> misc_feature
   <222> (22)..(22)
   <223> Xaa is Ornithine amide
<400> 54
   Gly Ala Arg Val Asp Lys Pro Pro Tyr Leu Pro Arg Pro Arg Pro Xaa
   1               5                   10                  15
   Arg Xaa Xaa Tyr Asn Xaa
               20
<210> 55
   <211> 22
   <212> PRT
   <213> Artificial Sequence
<220>
   <223> Artificial Peptide
<220>
   <221> misc_feature
   <222> (1)..(1)
   <223> Xaa is glycine with Polyethylene glycole 750 bound to N-terminus
<220>
   <221> misc_feature
   <222> (18)..(18)
   <223> Xaa is Ornithine
<220>
   <221> misc_feature
   <222> (22)..(22)
   <223> Xaa is Ornithine amide
<400> 55
   Xaa Ala Arg Val Asp Lys Pro Pro Tyr Leu Pro Arg Pro Arg Pro Pro
   1               5                   10                  15
   Arg Xaa Ile Tyr Asn Xaa
               20
<210> 56
   <211> 22
   <212> PRT
   <213> Artificial Sequence
<220>
   <223> Artificial Peptide
<220>
   <221> misc_feature
   <222> (1)..(1)
   <223> Xaa is glycine with Polyethylene glycole 750 bound to N-terminus
<220>
   <221> misc_feature
   <222> (16)..(16)
   <223> Xaa is 4-Hydroxyproline
<220>
   <221> misc_feature
   <222> (18)..(18)
   <223> Xaa is 4-Hydroxyproline
<220>
   <221> misc_feature
   <222> (19)..(19)
   <223> Xaa is tert-Leucine
<220>
   <221> misc_feature
   <222> (22)..(22)
   <223> Xaa is Ornithine amide
<400> 56
   Xaa Ala Arg Val Asp Lys Pro Pro Tyr Leu Pro Arg Pro Arg Pro Xaa
   1               5                   10                  15
   Arg Xaa Xaa Tyr Asn Xaa
               20
<210> 57
   <211> 22
   <212> PRT
   <213> Artificial Sequence
<220>
   <223> Artificial Peptide
<220>
   <221> misc_feature
   <222> (1)..(1)
   <223> Xaa is glycine with thiol modified Polyethylene glycole 750 bound to
   iodoacetylated N-terminus
<220>
   <221> misc_feature
   <222> (18)..(18)
   <223> Xaa is Ornithine
<220>
   <221> misc_feature
   <222> (22)..(22)
   <223> Xaa is Ornithine amide
<400> 57
   Xaa Ala Arg Val Asp Lys Pro Pro Tyr Leu Pro Arg Pro Arg Pro Pro
   1               5                   10                  15
   Arg Xaa Ile Tyr Asn Xaa
               20
<210> 58
   <211> 22
   <212> PRT
   <213> Artificial Sequence
<220>
   <223> Artificial Peptide
<220>
   <221> misc_feature
   <222> (1)..(1)
   <223> Xaa is glycine with thiol modified Polyethylene glycole 750 bound to iodoacetylated N-terminus
<220>
   <221> misc_feature
   <222> (16)..(16)
   <223> Xaa is 4-Hydroxyproline
<220>
   <221> misc_feature
   <222> (18)..(18)
   <223> Xaa is 4-Hydroxyproline
<220>
   <221> misc_feature
   <222> (19)..(19)
   <223> Xaa is tert-Leucine
<220>
   <221> misc_feature
   <222> (22)..(22)
   <223> Xaa is Ornithine amide
<400> 58
   Xaa Ala Arg Val Asp Lys Pro Pro Tyr Leu Pro Arg Pro Arg Pro Xaa
   1               5                   10                  15
   Arg Xaa Xaa Tyr Asn Xaa
               20
<210> 59
   <211> 22
   <212> PRT
   <213> Artificial Sequence
<220>
   <223> Artificial Peptide
<220>
   <221> misc_feature
   <222> (1)..(1)
   <223> Xaa is glycine with thiol modified Polyethylene glycole 5000 bound to iodoacetylated N-terminus
<220>
   <221> misc_feature
   <222> (18)..(18)
   <223> Xaa is Ornithine
<220>
   <221> misc_feature
   <222> (22)..(22)
   <223> Xaa is Ornithine amide
<400> 59
   Xaa Ala Arg Val Asp Lys Pro Pro Tyr Leu Pro Arg Pro Arg Pro Pro
   1               5                   10                  15
   Arg Xaa Ile Tyr Asn Xaa
               20
<210> 60
   <211> 22
   <212> PRT
   <213> Artificial Sequence
<220>
   <223> Artificial Peptide
<220>
   <221> misc_feature
   <222> (1)..(1)
   <223> Xaa is glycine with thiol modified Polyethylene glycole 5000 bound to iodoacetylated N-terminus
<220>
   <221> misc_feature
   <222> (16)..(16)
   <223> Xaa is 4-Hydroxyproline
<220>
   <221> misc_feature
   <222> (18)..(18)
   <223> Xaa is 4-Hydroxyproline
<220>
   <221> misc_feature
   <222> (19)..(19)
   <223> Xaa is tert-Leucine
<220>
   <221> misc_feature
   <222> (22)..(22)
   <223> Xaa is Ornithine amide
<400> 60
   Xaa Ala Arg Val Asp Lys Pro Pro Tyr Leu Pro Arg Pro Arg Pro Xaa
   1               5                   10                  15
   Arg Xaa Xaa Tyr Asn Xaa
               20
<210> 61
   <211> 5
   <212> PRT
   <213> Artificial Sequence
<400> 61
   Gly Arg Ser Gly
<210> 62
   <211> 22
   <212> PRT
   <213> Artificial Sequence
<400> 62
<210> 63
   <211> 6
   <212> PRT
   <213> Artificial Sequence
<400> 63
   Gly Ala Ala Arg Ser Gly
<210> 64
   <211> 7
   <212> PRT
   <213> Artificial Sequence
<400> 64
   Gly Ala Ala Ala Arg Ser Gly
                   5
<210> 65
   <211> 8
   <212> PRT
   <213> Artificial Sequence
<400> 65
   Gly Ala Ala Ala Ala Arg Ser Gly
                   5
<210> 66
   <211> 4
   <212> PRT
   <213> Artificial Sequence
<400> 66
   Gly Ala Ala Arg